# EUROPÄISCHE PATENTSCHRIFT

(11) **EP 2 969 028 B1**
(45) Veröffentlichungstag und Bekanntmachung des Hinweises auf die Patenterteilung: **09.05.2018**
(21) Anmeldenummer: 14704593.4
(22) Anmeldetag: 14.02.2014
(51) Int. Cl.: A61Q 19/02, A61Q 17/04, A61K 31/426, A61K 8/06

(54) **ZUSAMMENSETZUNGEN AUS ALKYLAMIDOTHIAZOLEN UND UV-FILTERSUBSTANZEN**
COMPOSITIONS OF ALKYLAMIDOTHIAZOLES AND UV FILTER SUBSTANCES
MÉLANGES CONTENANT ALKYLAMIDOTHIAZOLES ET SUBSTANCES DE FILTRE UV

(30) Priorität: 11.03.2013 DE 102013204097
(43) Veröffentlichungstag der Anmeldung: 20.01.2016
(73) Patentinhaber: Beiersdorf AG, 20253 Hamburg (DE)
(72) Erfinder: MANN, Tobias, 22175 Hamburg (DE); SCHERNER, Cathrin, 22844 Norderstedt (DE); KOLBE, Ludger, 21255 Dohren (DE); BATZER, Jan, 22459 Hamburg (DE)
(86) Internationale Anmeldenummer: PCT/EP2014/052972
(87) Internationale Veröffentlichungsnummer: WO 2014/139759

(56) Entgegenhaltungen:
- WO-A2-2011/117034

## Beschreibung

Die vorliegende Erfindung betrifft Wirkstoffkombinationen aus Alkylamidothiazolen und einer oder mehreren kosmetisch oder dermatologisch unbedenklichen UV-Filtersubstanzen. Weiterhin betrifft die vorliegende Erfindung kosmetische oder dermatologische Zubereitungen mit einem Gehalt an solchen Wirkstoffkombination sowie deren Verwendung zum Aufhellen der menschlichen Haut.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. - vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte "Triformula" entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Weiterhin sind diverse andere Substanzen bekannt, für die eine hautaufhellende Wirksamkeit beschrieben wird. U.a. zu nennen sind hier Hexadecen-1,16-dicarbonsäure, Kojic-Säure und Derivate, Arbutin, Ascorbinsäure und Derivate, Flavonoide, Ellagsäure und Derivate, Tranexamsäure und verschiedene Resorcinol-Derivate, wie z.B. 4-n-Butylresorcin, 4-n-Hexylresorcin und 4-(1-phenylethyl)benzen-1,3-diol.

J.M. Ready beschreibt in einer Publikation (Bioorganic & Medicinal Chemistry Letter 17 (2007) 6871-6875 die Wirkung von u.a. substituierten Thiazol-Derivaten zur Inhibition der Mushroom tyrosinase.

In der Patentanmeldung der Firma Shiseido (WO 2009099195) werden substituierte Thiazolamine bzw. Hydrothiazolamine zur Hautaufhellung beschrieben. Alkylamidothiazolverbindungen zur dermatologischen Anwendung finden sich in der WO2011/117034. Die im oben genannten Stand der Technik beschriebenen Substanzen weisen sich durch eine moderate Wirksamkeit aus.

Augenringe können ebenfalls als Folgen einer Pigmentierungsstörung entstehen, wobei sie ferner auch als Reaktion auf allgemeinen Stress, wie z.B. wenig Schlaf oder schlicht durch Überanstrengung der Augen erscheinen. Bei jüngeren Menschen verschwinden die Symptome nach ausreichender Nachtruhe wieder, über längere Zeiträume jedoch kann der Zustand chronisch und für die betroffenen Personen sehr störend werden. Auch gegen solche Hauterscheinungen mangelt es an genügend erfolgversprechenden Wirkstoffen und Behandlungsmöglichkeiten.

Ziel der nachfolgenden Erfindung war es also, dem nachteiligen Stand der Technik Abhilfe zu schaffen.

Gelöst wird diese Aufgabe durch Wirkstoffkombinationen aus einem oder mehreren Alkylamidothiazolen und einer oder mehreren UV-Filtersubstanzen wobei die UV-Filtersubstanz oder die UV-Filtersubstanzen gewählt wird oder werden aus der Gruppe Butylmethoxydibenzoylmethan, Ethylhexylmethoxycinnamat, Octocrylen, Ethylhexylsalicylat, Phenylbenzimidazolsulfonsäure, Dinatriumphenyldibenzimidazoltetrasulfonat, Benzophenon-3, Drometrizol trisiloxan, Benzophenon-4, Homosalate, Benzol-1,4-di(2-oxo-3-bornylidenme-thyl-10-sulfonsäure, Polysilicone-15, Ethylhexyl Triazone, Diethylhexyl-Butamidotriazone, **Isoamyl p-Methoxycinnamate, Diethylamino Hydroxybenzoyl Hexyl Benzoate,** Methylenbis-benzotriazolyltetramethylbutylphenol, Bis-Ethylhexyloxyphenolmethoxyphenyltriazin, 4-Methylbenzylidencampher, 2,4-Bis-[5-](dimethylpropyl)benzoxazol-2-yl-(4-phenyl)-imino]-6-(2-ethylhexyl)-imino-1,3,5-triazin und/oder (2-{4-[2-(4-Diethylamino-2-hydroxy-benzoyl)-benzoyl]-piperazin-1-carbonyl}-phenyl)-(4-diethylamino-2-hydroxy-phenyl)-methanon, dadurch gekennzeichnet, daß das oder die Alkylamidothiazole folgende Struktur aufweisen: 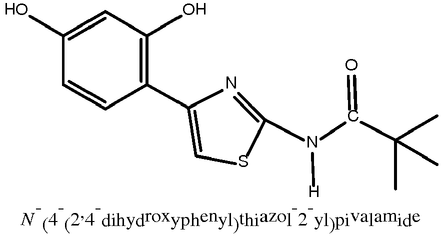 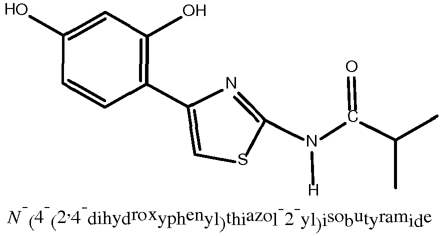 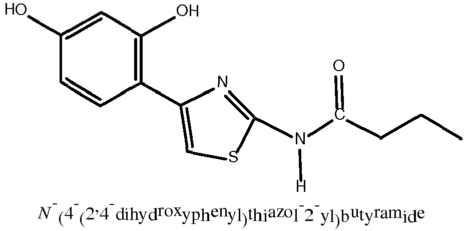 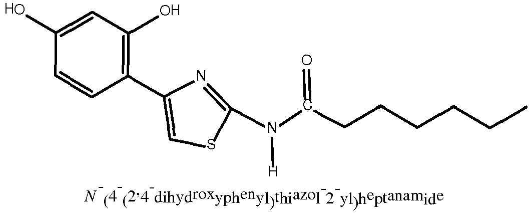 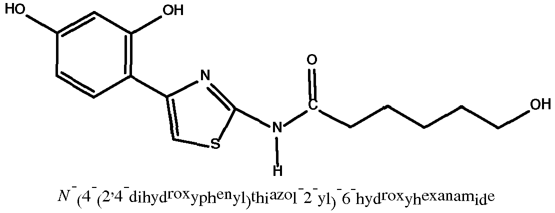 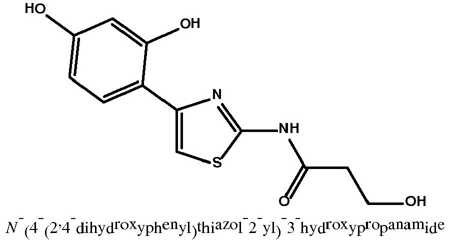 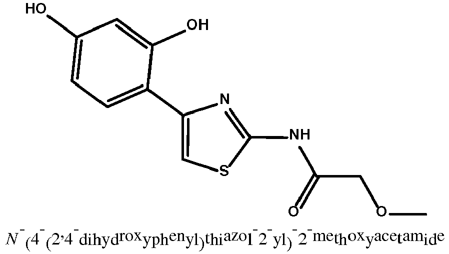 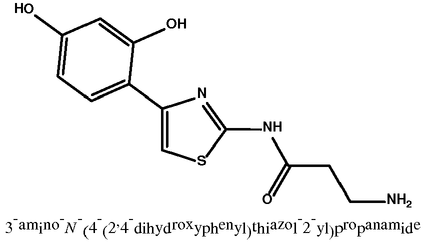 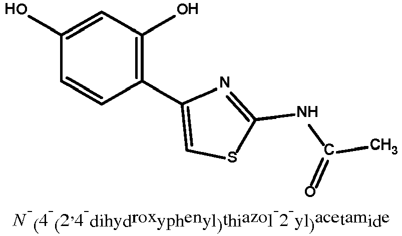 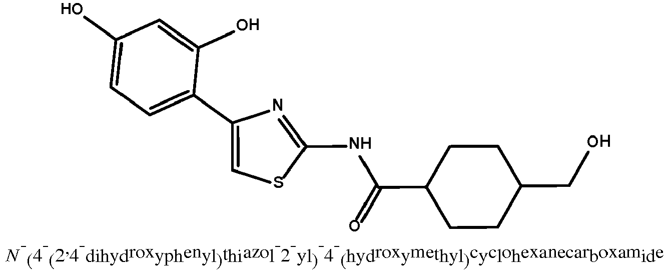 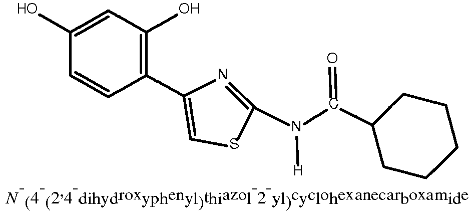 und

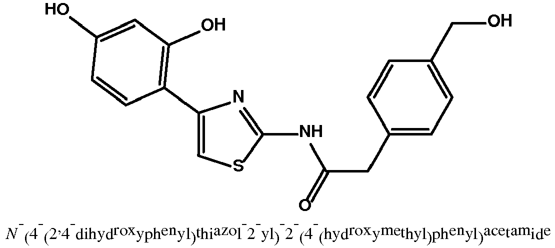

Vorteilhafte Verkörperungen der vorliegenden Erfindung sind auch kosmetische oder dermatologische Zubereitungen mit einem Gehalt an solchen Wirkstoffkombination sowie deren Verwendung zum Aufhellen der menschlichen Haut.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,01 Gew.-% bis 30 Gew.-%, vorzugsweise 0,05 bis 20 Gew.-%, insbesondere 0,1 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhaft enthalten die erfindungsgemäßen Zubereitungen 0,01 - 30 Gew-% an einer oder mehreren UV-Licht absorbierenden Substanzen, vorzugsweise 0,05 - 20 Gew-% an an einer oder mehreren UV-Licht absorbierenden Substanzen, insbesondere bevorzugt 0,1 -15 Gew-% an einer oder mehreren UV-Licht absorbierenden Substanzen.

Vorteilhaft sind insbesondere erfindungsgemäße Zubereitungen bzw. Verwendungen, dadurch gekennzeichnet, daß die Zubereitungen 0,000001 bis 10 Gew.-%, insbesondere 0,0001 bis 3 Gew.-%, ganz besonders 0,001 bis 1 Gew.-% an an einem oder mehreren Alkylamidothiazolen enthalten, bezogen auf das Gesamtgewicht der Zubereitung.

Überraschenderweise konnte gezeigt werden, daß die erfindungsgemäßen Alkylamidothiazole in Kombination mit erfindungsgemäßen UV-Filtern eine gesteigerte Wirksamkeit aufweisen.

### Methodenbeschreibung der Wirksamkeitsuntersuchungen:

Die Wirksamkeit der Thiazole wurde mit einem Enzymtest belegt, in der Umsatz von L-DOPA zu L-Dopachinon durch eine humane Tyrosinase gemessen wurde. Bei dieser literaturbekannten Methode (Winder, A.J. and Harris, H., New assays for the tyrosine hydroxylase and dopa oxidase activities of tyrosinase. Eur. J. Biochem. (1991), 198, 317-26) wird das Reaktionsprodukt L-Dopaquinone mit MBTH (3-methyl-2-benzothiazoline hydrazone) zu einer pinkfarbenen Substanz umgesetzt, deren Zunahme über die Zeit durch Absorption bei 490 nm gemessen wird. In Tabelle ein sind Wirksamkeitsdaten für einige der beanspruchten Substanzen beispielhaft dargestellt. Daraus lässt sich schließen, dass die erfindungsgemäßen Substanzen äußerst effektive pigmentierungs-inhibierende Substanzen sind.

| **Tabelle: Inhibition der Tyrosinaseaktivität durch die Kombination von N-(4-(2,4-Dihydroxyphenyl)thiazol-2-yl)-isobutyramid mit verschiedenen UV-Filtern** | | |
|---|---|---|
| *Substanz* | *Inhibition (% der Kontrolle)* | *Konzentration* |
| N-(4-(2,4-Dihydroxyphenyl)thiazol-2-yl)-isobutyramid | 33,5 | 0,3 µg/mL |
| Ethylhexyl Salicylat | 8,7 | 72 µg/mL |
| N-(4-(2,4-Dihydroxyphenyl)thiazol-2-yl)-isobutyramid + Ethylhexyl Salicylat | 43,2 | 72,3 µg/mL |
| N-(4-(2,4-Dihydroxyphenyl)thiazol-2-yl)-isobutyramid | 33,5 | 0,3 µg/mL |
| Isoamyl p-Methoxycinnamat | 32,8 | 54 µg/mL |
| N-(4-(2,4-Dihydroxyphenyl)thiazol-2-yl)-isobutyramid + Isoamyl p-Methoxycinnamat | 41,5 | 54,3 µg/mL |
| N-(4-(2,4-Dihydroxyphenyl)thiazol-2-yl)-isobutyramid | 33,5 | 0,3 µg/mL |
| Phenylbenzimidazole Sulfonsäure | 0,7 | 48 µg/mL |
| N-(4-(2,4-Dihydroxyphenyl)thiazol-2-yl)-isobutyramid + Phenylbenzimidazol Sulfonsäure | 45,1 | 48,3 µg/mL |
| N-(4-(2,4-Dihydroxyphenyl)thiazol-2-yl)-isobutyramid | 33,5 | 0,3 µg/mL |
| Benzophenon-4 | 45,0 | 150 µg/mL |
| N-(4-(2,4-Dihydroxyphenyl)thiazol-2-yl)-isobutyramid + Benzophenon-4 | 50,8 | 150,3 µg/mL |

### Synthesevorschriften exemplarisch ausgewählter Alkylamidothiazole:

### 2-Brom-2',4'-bis-methoxycarbonyloxy-acetophenon:

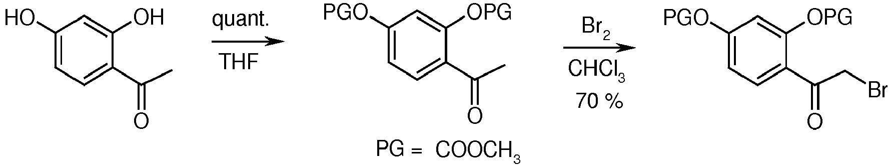

Mitchell, David; Doecke, Christopher W.; Hay, Lynne A.; Koenig, Thomas M.; Wirth, David D. Tetrahedron Letters, 1995
Eine Lösung von 60g (369 mmol) 2,4-Dihydroxyacetophenon and 186 ml Triethylamin in 900 ml Tetrahydrofuran wurde auf 0°C gekühlt und 93 ml Chlorameisensäuremethylester in 400 ml Tetrahydrofuran langsam zugetropft. Es bildet sich ein weißer Niederschlag. Nach 3 Stunden Rühren bei Raumtemperatur ist die Reaktion abgeschlossen (DC-Kontrolle). Der Niederschlag wurde abgesaugt und mit reichlich Tetrahydrofuran gewaschen. Das Filtrat wurde zur Trockne einrotiert, in Ethylacetat aufgenommen mit 1N HCl und NaCl-Lösung (sat.) gewaschen und über Magnesiumsulfat getrocknet, vom Magnesiumsulfat filtriert und das Ethylacetat am Rotationsverdampfer eingeengt. Es wurden 105 g von 2,4-Bis-methoxycarbonyloxy-acetophenon erhalten. ¹H NMR (DMSO-D₆): 8.05 (d, 1H), 7.38 (d, 1H), 7.36 (s, 1H), 3.86 (d, 6H). Das Produkt wurde ohne weitere Reinigung eingesetzt. Zu der Lösung von 105 g 2,4-Bis-methoxycarbonyloxy-acetophenon in Chloroform (1000 ml) wurden 63g (392 mmol) Brom in 450 ml Chloroform innerhalb von 3 h zugetropft. Danach wurde die Reaktion noch 15 min. bei Raumtemperatur gerührt, Das Lösungsmittel wurde einrotiert. Der Rückstand wurde in Ethylacetat/n-Hexan verrührt, der entstandene Niederschlag wurde abgesaugt. Umkristallisation aus Ethylacetat/n-Hexan lieferten 100 g 2-Brom-2',4'- bis-methoxycarbonyloxy-acetophenon. ¹H NMR (DMSO-D₆): 8.11 (d, 1H), 7.42 (m, 2H), 4. 87 (s, 2H), 3.87 (s, 3H), 3.85 (s, 3H) ppm; m.p. 73-74°C.

### N-(4-(2,4-Dihydroxyphenyl)thiazol-2-yl)-pivalamid:

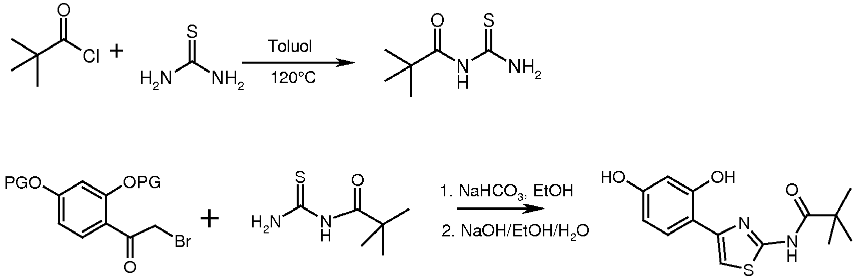

126 g (1.66 mmol) Thioharnstoff wurden in Toluol (1000 ml) vorlegt und 100 g (829 mmol) Pivaloylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen farblosen Nadeln wurden abgesaugt und mit Cyclohexan gewaschen und im Vakuum getrocknet. Ausbeute: 64 g. ¹H NMR (DMSO-D₆): 10.27 (s, 1H), 9.74 (s, 1H), 9.40 (s, 1H), 1.19 (s, 9H) ppm.

107.7 g (310 mmol) 2-Brom-2',4'- bis-methoxycarbonyloxy-acetophenon wurden mit 49.7 g (13.6 mmol) N-Pivaloylthioharnstoff und 39.2 g (466 mmol) NaHCO₃ in 1.2l Ethanol unter Rückfluss für 0.5h gekocht. Die Reaktionslösung wurde abgekühlt und mit 50.6 g (1.27 mol) NaOH in 250 ml Wasser versetzt. Nach 30 Min. Rühren bei Raumtemperatur wurde die Reaktionslösung mit 300 ml Wasser aufgenommen und mit 2N HCl neutralisiert. Der entstandene Niederschlag wurde abfiltriert und aus Ethanol/Wasser umkristallisiert. Es wurden 80 g Thiazol erhalten. ¹H NMR (DMSO-D₆): 11.77 (bs, 1H), 11.02 (bs, 1H), 9.47 (bs, 2H), 7.65 (d, 1H), 7.39 (s, 1H), 6.30 (s, 1H), 6.28 (d, 1H), 1.27 (s, 9H) ppm; m.p. 257-259°C.

### N-(4-(2,4-Dihydroxyphenyl)thiazol-2-yl)-isobutyramid:

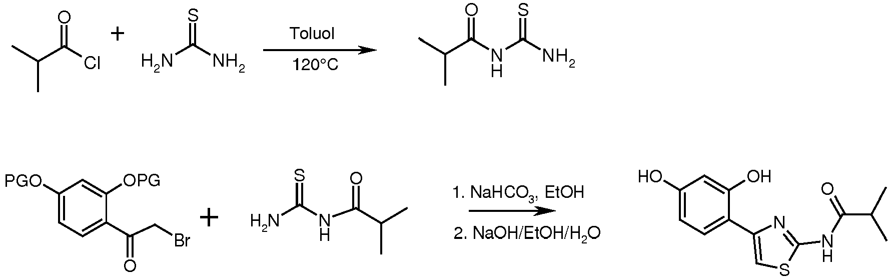

114 g (1.5 mol) Thioharnstoff wurden in Toluol (800 ml) vorgelegt und 80 g (0.75 mol) Isobutyrylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen weißen Kristalle wurden abgesaugt und mit Toluol gewaschen und im Vakuum getrocknet. Ausbeute: 62 g. ¹H NMR (DMSO-D₆): 11.03 (bs, 1H), 9.66 (bs, 1H), 9.35 (bs, 1H), 2.72 (m, 1H), 1.03 (2, 6H) ppm;

89 g (260 mmol) 2-Brom-2',4'-bis-methoxycarbonyloxy-acetophenon wurden mit 37.5 g (260 mmol) N-Isobutyrylthioharnstoff und 32 g (380 mmol) NaHCO₃ in 1000 ml Ethanol unter Rückfluss für 0.5h gekocht. Die Reaktionslösung wurde abgekühlt und mit 41 g (0.93 mol) NaOH in 250 ml Wasser versetzt. Nach 30 Min. Rühren bei Raumtemperatur wurde die Reaktionslösung mit 300 ml Wasser aufgenommen und mit 2N HCl auf pH=3 eingestellt. Der entstandene Niederschlag wurde abfiltriert und aus Ethanol/Wasser umkristallisiert. Es wurden 56 g Thiazol erhalten. ¹H NMR (DMSO-D₆): 12.16 (bs, 1H), 10.88 (bs, 1H), 9.47 (bs, 1H), 7.65 (m, 1H), 7.41 (s, 1H), 6.32 (m, 2H), 2.75 (m, 1H), 1.14 (d, 6H) ppm. m.p.: 243-245°C.

### N-(4-(2,4-Dihydroxyphenyl)thiazol-2-yl)-butyramid:

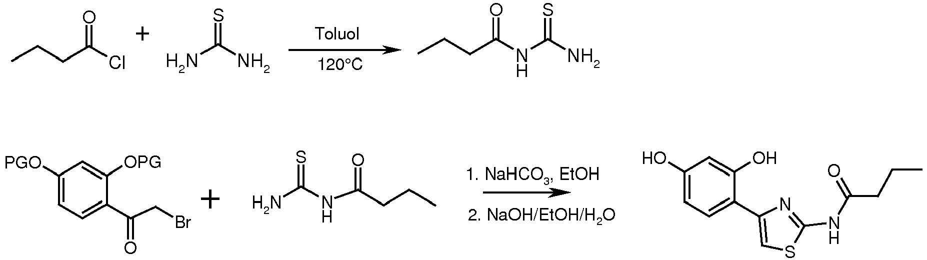

143 g (1.88 mol) Thioharnstoff wurden in Toluol (1000 ml) vorgelegt und 100 g (0.93 mol) n-Butyrylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen leicht gelblichen Kristalle wurden abgesaugt und mit Toluol gewaschen und im Vakuum getrocknet. Ausbeute: 88 g. ¹H NMR (DMSO-D₆): 11.03 (bs, 1H), 9.65 (bs, 1H), 9.33 (bs, 1H), 2.33 (t, 2H), 1.53 (m, 2H), 0.86 (t, 3H) ppm; m.p.: 115-188°C

92 g (265 mmol) 2-Brom-2',4'- bis-methoxycarbonyloxy-acetophenon wurden mit 38.75 g (265 mmol) N-Butyrylthioharnstoff und 34 g (397 mmol) NaHCO₃ in 900 ml Ethanol unter Rückfluss für 0.5h gekocht. Die Reaktionslösung wurde abgekühlt und mit 37 g (0.93 mol) NaOH in 300 ml Wasser versetzt. Nach 30 Min. Rühren bei Raumtemperatur wurde die Reaktionslösung mit 300 ml Wasser aufgenommen und mit 2N HCl neutralisiert. Der entstandene Niederschlag wurde abfiltriert und aus Ethanol/Wasser umkristallisiert. Es wurden 67 g Thiazol erhalten. ¹H NMR (DMSO-D₆): 12.18 (bs, 1H), 10.89 (bs, 1H), 9.48 (bs, 1H), 7.65 (1 arom. H), 7.40 (s, 1H), 6.31 (2 arom. H), 2.43 (t, 2H), 1.64 (m, 2H), 0.91 (t, 3H)ppm. m.p.: 227-229°C.

### N-(4-(2,4-Dihydroxyphenyl)thiazol-2-yl)-acetamid:

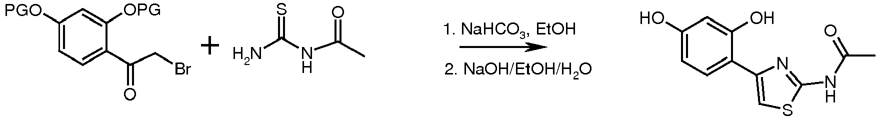

4.71 g (13.6 mmol) von 2-Brom-2',4'- bis-methoxycarbonyloxy-acetophenon wurden mit 1.61 g (13.6 mmol) N-Acetylthioharnstoff und 1.72 g (20.4 mmol) NaHCO₃ in 45 ml Ethanol 0.5h unter Rückfluss gekocht. Die Reaktionslösung wurde abgekühlt und mit 2.0 g (50 mmol) NaOH in 20 ml Wasser versetzt. Nach 20 Min. Rühren bei 0°C wurde die Reaktionslösung mit 30 ml Wasser aufgenommen und mit halbkonz. HCl neutralisiert. Der entstandene Niederschlag wurde abfiltriert und aus Ethanol/Wasser umkristallisiert. Es wurden 2.73g Produkt erhalten. ¹H NMR (DMSO-D₆): 12.20 (b, 1H), 10.85 (s, 1H), 9.46 (s, 1H), 7.64 (m, 1H), 7.38 (s, 1H), 6.28 (m, 2H), 2.15 (s, 3H) ppm; m.p. 264-264°C.

### N-(4-(2,4-Dihydroxyphenyl)thiazol-2-yl)4-(Hydroxymethyl)cyclohexancarboxamid:

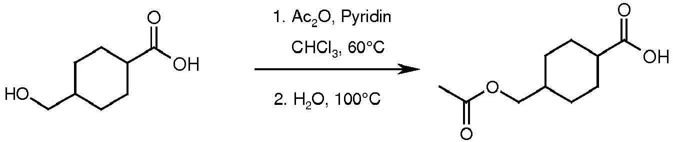

Durchführung analog Literatur.
BANYU Pharmaceutical Co., Ltd., EP2072519 A1, 2009
Ausbeute: 96%, ¹H NMR (DMSO-D₆): 12.03 (bs, 1H), 3.85, 3.82 (2 x d, 2H), 2.50, 2.47 (2 x m, 1H), 2.00 (s, 3H), 0.95-1.90 (m, 9H) ppm;

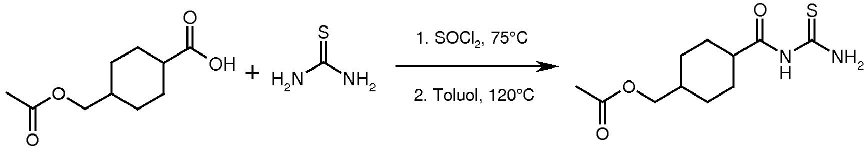

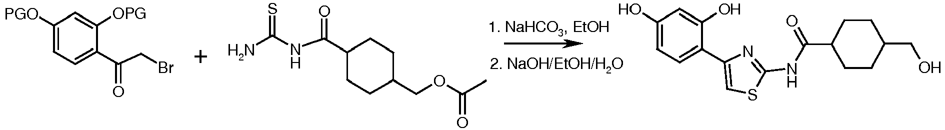

95 g (0.47 mol) 4-Acetoxymethylcyclohexancarbonsäure wurden in 350 ml Thionylchlorid 2h unter Rückfluss erhitzt. Nach Entfernen des überschüssigen Thionylchlorids im Vakuum wurde der Rückstand in 1l Toluol aufgenommen und 71 g (0.94 mol) Thioharnstoff zugegeben. Die Reaktionslösung wurde 3 Stunden unter Rückfluss gekocht und anschließend heiß filtriert. Nach Abkühlen der Mutterlauge wurden die entstandenen weißen Kristalle abgesaugt, mit Toluol gewaschen und im Vakuum getrocknet. Ausbeute: 59 g. ¹H NMR (DMSO-D₆): 11.03, 10.97 (2 x s, 1H), 9.64 (bs, 1H), 9.35 (bs, 1H), 3.93, 3.82 (2 x d, 2H), 2.61, 2.42 (2 x m, 1H), 2.00 (s, 3H), 1.60 (m, 8H), 1.35, 0.94 (2 x m, 1H) ppm;

79 g (228 mmol) 2-Brom-2',4'- bis-methoxycarbonyloxy-acetophenon wurden mit 59 g (228 mmol) N-(4-Acetoxymethylcyclohexylcarbonyl)thioharnstoff und 29 g (340 mmol) NaHCO₃ in 1000 ml Ethanol unter Rückfluss für 0.5h gekocht. Die Reaktionslösung wurde abgekühlt und mit 73 g (1.8 mol) NaOH in 300 ml Wasser versetzt. Nach 30 Min. Rühren bei Raumtemperatur wurde die Reaktionslösung mit 300 ml Wasser aufgenommen und mit 2N HCl auf pH=3 eingestellt. Der entstandene Niederschlag wurde abfiltriert und aus Ethanol/Wasser umkristallisiert. Es wurden 47 g Thiazol erhalten. ¹H NMR (DMSO-D₆): 12.15, 12.10 (2 x s, 1H), 10.96 (2 x s, 1H), 9.47 (br, 2H), 7.64 (d, 1H), 7.39 (s, 1H), 6.29 (m, 2H), 4.40 (br, 1H), 3.32, 3.23 (2 x d, 2H), 2.65, 2.44 (2 x m, 1H), 1.90 (m, 1H), 1.78 (m, 2H), 1.50 (m, 5H), 0.94 (m, 1H) ppm. m.p.: 152-160°C.

### N-(4-(2,4-Dihydroxyphenyl)thiazol-2-yl)-cyclohexacarboxamid:

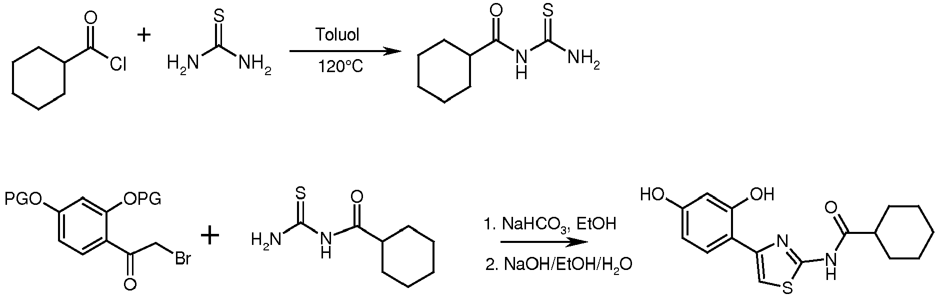

52 g (0.68 mol) Thioharnstoff wurden in Toluol (500 ml) vorgelegt und 50 g (0.34 mol) Cyclohexanoylchlorid zugetropft. Die Reaktionslösung wurde 3 Stunden unter Rückfluss gekocht, wobei 2 Phasen entstehen. Die obere Phase wurde abdekantiert und abgekühlt. Die ausgefallenen Kristalle wurden abgesaugt, mit Toluol gewaschen und aus Methanol umkristallisiert. Ausbeute: 35 g. ¹H NMR (DMSO-D₆): 10.98 (bs, 1H), 9.65 (bs, 1H), 9.32 (bs, 1H), 2.49 (t, 1H), 1.75 (m, 4H), 1.61 (m, 1H), 1.18 (m, 5H) ppm.

92 g (265 mmol) 2-Brom-2',4'- bis-methoxycarbonyloxy-acetophenon wurden mit 49.4 g (265 mmol) N-Cyclohexanoylthioharnstoff und 34 g (397 mmol) NaHCO₃ in 900 ml Ethanol unter Rückfluss für 0.5h gekocht. Die Reaktionslösung wurde abgekühlt und mit 37 g (930 mmol) NaOH in 300 ml Wasser versetzt. Nach 30 Min. Rühren bei Raumtemperatur wurde die Reaktionslösung mit 300 ml Wasser aufgenommen und mit 2N HCl neutralisiert. Das Ethanol wurde weitgehend am Rotationsverdampfer entfernt. Der entstandene Niederschlag wurde abfiltriert und aus Ethanol/Wasser umkristallisiert. Es wurden 70 g Thiazol erhalten. ¹H NMR (DMSO-D₆): 12.14 (bs, 1H), 11.00 (bs, 1H), 9.48 (bs, 1H), 7.64 (1 arom. H), 7.39 (s, 1H), 6.30 (2 arom. H), 2.49 (m, 1H), 1.84 (m, 2H), 1.76 (m, 2H), 1.65 (m, 1H), 1.42 (m, 2H), 1.25 (m, 3H) ppm. m.p.: 262-266°C.

### N-(4-(2,4-dihydroxyphenyl)thiazol-2-yl)-2-(4-(hydroxymethyl)phenyl)acetamid:

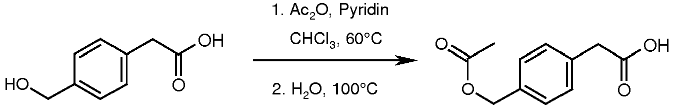

Durchführung analog Literatur.
BANYU Pharmaceutical Co., Ltd., EP2072519 A1, 2009
Ausbeute: 76%, ¹H NMR (DMSO-D₆): 12.31 (bs, 1H), 7.26 (m, 4H), 5.05 (s, 2H), 3.57 (s, 2H), 2.05 (s, 3H) ppm;

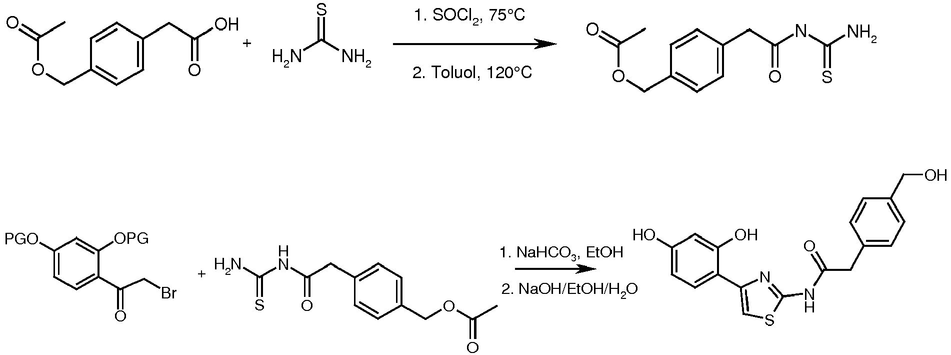

3.7 g (18 mmol) 4-Acetoxymethylphenylessigsäure wurden in 40 ml Thionylchlorid 2 h unter Rückfluss erhitzt. Nach Entfernen des überschüssigen Thionylchlorids im Vakuum wurde der Rückstand in 70 ml Toluol aufgenommen und 2.7 g (36 mmol) Thioharnstoff zugegeben. Die Reaktionslösung wurde 3 Stunden unter Rückfluss gekocht und anschließend wurde das Lösungsmittel im Vakuum entfernt. Die Reinigung erfolgte mittels Säulenchromatographie mit Cyclohexan/Essigester 1/1 an Kieselgel. Ausbeute: 2.7 g. ¹H NMR (DMSO-D₆): 11.29 (bs, 1H), 9.55 (bs, 1H), 9.40 (bs, 1H), 7.30 (m, 4H), 5.04 (s, 2H), 3.71 (s, 2H), 2.05 (s, 3H) ppm;

3.5 g (10 mmol) 2-Brom-2',4'- bis-methoxycarbonyloxy-acetophenon wurden mit 2.7 g (10 mmol) N-[2-(4-Acetoxymethylphenyl)acetyl]thioharnstoff und 1.3 g (15 mmol) NaHCO₃ in 50 ml Ethanol unter Rückfluss für 0.5h gekocht. Die Reaktionslösung wurde abgekühlt und mit 4.0 g (0.1 mol) NaOH in 20 ml Wasser versetzt. Nach 2 h Rühren bei 60°C wurde die Reaktionslösung in 100 ml Wasser aufgenommen und mit 2N HCl auf pH=3 eingestellt. Der entstandene Niederschlag wurde abfiltriert und aus Ethanol/Wasser umkristallisiert. Es wurden 1.3 g Thiazol erhalten. ¹H NMR(DMSO-D₆): 12.44 (s, 1H), 10.80 (s, 1H), 9.48 (s, 1H), 7.66 (d, 1H), 7.41 (s, 1H), 7.29 (m, 4H), 6.32 (m, 2H), 5.13 (t, 1H), 4.47 (d, 2H), 3.77 (s, 2H) ppm. m.p.: 254-256°C.

Kosmetische oder dermatologische Zubereitungen mit einem Gehalt an Alkylamidothiazolen bzw. deren Verwendung zur Behandlung und/oder Prophylaxe unerwünschter Hautpigmentierung, sind ebenfalls vorteilhafte Verkörperungen der vorliegenden Erfindung.

Vorteilhaft ist es insbesondere, wenn solche Zubereitungen 0,000001 bis 10 Gew.-%, insbesondere 0,0001 bis 3 Gew.-%, ganz besonders 0,001 bis 1 Gew.-% an einem oder mehreren der erfindungsgemäß verwendeten Alkylamidothiazolen enthalten, bezogen auf das Gesamtgewicht der Zubereitung.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindungsgemäß vorteilhaft, die erfindungsgemäß verwendeten Substanzen und/oder deren Derivate in verkapselter Form darzureichen, z.B. in Kollagenmatrices und anderen üblichen Verkapselungsmaterialien, z.B. als Celluloseverkapselungen, in Gelatine oder liposomal verkapselt.

Es ist auch möglich und vorteilhaft im Sinne der vorliegenden Erfindung, die erfindungsgemäß verwendeten Substanzen und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Feuchthaltemittel wie z.B. PropylenGlykol, Panthenol oder Hyaluronsäure sowie insbesondere ein oder mehrere Verdickungsmittel, welches oder welche vorteilhaft gewählt werden können aus der Gruppe Siliciumdioxid, Aluminiumsilikate, Hydroxypropylmethylcellulose, besonders vorteilhaft ein Polyacrylat wie beispielsweise Carbopole Typ 980, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, PropyleGlykol, und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Außerdem können vorteilhaft erfindungsgemäße Zubereitungen weiterhin Substanzen enthalten, die zur Konservierung dienen, wobei die Gesamtmenge der Konservierungsmittel z.B. 0,001 Gew.-% bis 30 Gew.-%, vorzugsweise 0,05 bis 10 Gew.-%, insbesondere 0,1 bis 5,0 Gew.-% beträgt, bezogen auf das Gesamtgewicht der Zubereitungen, um kosmetische Zubereitungen zur Verfügung zu stellen.

Desweiteren können vorteilhaft erfindungsgemäße Zubereitungen zusätzlich Substanzen enthalten, die störenden Eigengeruch der restlichen verwendeten Rohstoffe überdecken, wobei die Gesamtmenge der Parfüminhaltsstoffe z.B. 0,001 Gew.-% bis 30 Gew.-%, vorzugsweise 0,05 bis 10 Gew.-%, insbesondere 0,1 bis 5,0 Gew.-% beträgt, bezogen auf das Gesamtgewicht der Zubereitungen, um kosmetische Zubereitungen zur Verfügung zu stellen. Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Rezepturbeispiele

### O/W -Emulsionen

| **Rezepturbeispiel** | **1** | **2** | **3** | **4** |
|---|---|---|---|---|
| **Chemische/INCI-Bezeichnung** | **Gew.-%** | **Gew.-%** | **Gew.-%** | **Gew.-%** |
| Stearinsäure | 2,50 | 2,00 | 2,00 | 2,50 |
| Glyceryl Stearat | 1,00 | 1,00 | 1,00 | 1,00 |
| C12-15 Alkyl Benzoat | 3,00 | 5,00 | 3,00 | 2,00 |
| Caprylsäure/Caprinsäure Triglycerid | 2,50 | 2,50 | 2,00 | 2,50 |
| Isopropyl Palmitat | 2,00 | - | - | 2,00 |
| Cetylstearylalkohol | 3,00 | - | 2,00 | 3,00 |
| Cetyl Alkohol | - | 2,00 | - | - |
| Stearyl Alkohol | - | 2,00 | 1,00 | - |
| C13-16 Isoparaffin | - | - | - | 1,00 |
| Dibutyladioat | - | - | 1,50 | - |
| Cyclomethicon | 1,00 | 1,00 | 0,50 | - |
| Dicaprylyl Carbonat | 2,00 | 2,00 | 2,00 | 2,00 |
| Dimethicon | 1,00 | - | 0,50 | 1,00 |
| Glycerin | 5,00 | 7,00 | 5,00 | 9,00 |
| Ethylhexyl Cocoat | - | - | 1,00 | - |
| Methylparaben | 0,20 | - | - | - |
| Phenoxyethanol | 0,40 | 0,50 | 0,50 | 0,40 |
| Propylparaben | 0,10 | - | - | 0,10 |
| 1,2-Hexandiol | - | - | 0,10 | 0,10 |
| Ethylhexylglycerin | - | - | 0,20 | - |
| Methylisothiazolinon | - | 0,05 | - | - |
| Butylenglykol | - | - | 2,0 | - |
| Carbomer | 0,15 | 0,10 | 0,15 | 0,10 |
| Carrageenan | 0,10 | - | 0,10 | - |
| Xanthan Gummi | - | - | 0,10 | - |
| Acrylates/C10-30 Alkyl Acrylate Crosspolymer | - | 0,10 | - | 0,10 |
| Trinatrium EDTA | 0,20 | 0,20 | 0,20 | 0,20 |
| Tapioka Stärke | 1,50 | 1,00 | - | |
| Nylon-12 (1,8-Diazacyclotetradecan-2,7-dion Homopolymer) | - | 0,20 | - | 0,50 |
| Polymethylsilsesquioxane | - | 1,00 | 1,00 | - |
| Aluminum Stärke Octenylsuccinat | - | - | 1,00 | - |
| Distärkephosphat | 1,00 | 1,00 | - | 1,00 |
| Butyl Methoxydibenzoylmethan | 1,00 | 2,00 | 1,00 | 1,00 |
| Phenylbenzimidazolsulfonsäure | 1,00 | 1,00 | 2,00 | 2,00 |
| Octocrylen | 2,00 | 2,00 | 1,00 | 2,00 |
| Ethylhexyl Salicylat | 1,00 | 1,00 | 2,00 | 1,00 |
| Ethylhexyl Triazone | 0,50 | 1,00 | 0,50 | 1,00 |
| Homosalat | 1,00 | 0,50 | 1,00 | 0,50 |
| Benzophenon-4 | 0,50 | 0,50 | 0,50 | 0,50 |
| (2-{4-[2-(4-Diethylamino-2-hydroxybenzoyl)-benzoyl]-piperazin-1-carbonyl}-phenyl)-(4-diethylamino-2-hydroxy-phenyl)-methanon | 0,50 | 0,50 | 0,50 | 0,50 |
| Hydroxypropyl tetrahydropyrantriol | 1,00 | 0,50 | - | - |
| Liponsäure | - | 0,50 | 0,20 | - |
| Kalium-Methoxysalicylate | 0,30 | - | 0,10 | 0,05 |
| Vitamin B6 HCl | 0,10 | 0,05 | - | 0,30 |
| Tranexamsäure | - | 0,01 | 0,25 | - |
| Pyrus Malus Stem Extract | 1,00 | 0,25 | 0,50 | 0,75 |
| N-(4-(2,4-Dihydroxyphenyl)thiazol-2-yl)-isobutyramid | 0,20 | 0.10 | 0.05 | 0.30 |
| N-(4-(2,4-Dihydroxyphenyl)thiazol-2-yl)-pivalamid | 0.01 | 0.25 | 0.15 | 0.10 |
| N-(4-(2,4-Dihydroxyphenyl)thiazol-2-yl)-butyramid | 0.25 | 0.15 | 0.30 | 0.35 |
| N-(4-(2,4-Dihydroxyphenyl)thiazol-2-yl)-cyclohexancarboxamid | 0.10 | 0.10 | 0.15 | 0.20 |
| Natriumhydroxid | q.s. | q.s. | q.s. | q.s. |
| Hydroxyisohexyl 3-Cyclohexencarboxaldehyd | 0,10 | - | - | 0,05 |
| Citronellol | 0,05 | 0,10 | - | 0,05 |
| Linalool | - | 0,05 | 0,10 | - |
| Parfüm | 0,30 | 0,20 | 0,20 | 0,20 |
| Natriumhydroxid | q.s. | q.s. | q.s. | q.s. |
| Wasser | ad 100 | ad 100 | ad 100 | ad 100 |

| **Rezepturbeispiel** | **5** | **6** | **7** | **8** |
|---|---|---|---|---|
| **Chemische Bezeichnung** | **Gew.-%** | **Gew.-%** | **Gew.-%** | **Gew.-%** |
| Glyceryl Stearat Citrat | 2,00 | 1,50 | 2,00 | 2,00 |
| Behenyl Alkohol | 1,50 | 1,00 | 1,00 | 1,00 |
| C12-15 Alkyl Benzoat | 2,00 | 2,50 | 2,00 | 2,50 |
| Caprylsäure/Caprinsäure Triglycerid | 2,00 | 2,00 | 2,50 | 2,50 |
| Cetyl Alkohol | 2,00 | 2,00 | - | 2,00 |
| Cetylstearylalkohol | - | - | 2,00 | - |
| Cyclopentasiloxane | - | - | - | 1,00 |
| Cyclomethicon | 1,00 | 1,00 | 2,00 | 2,00 |
| Dicaprylyl Carbonat | - | 2,00 | 2,50 | 2,50 |
| Paraffinum Liquidum (Mineralöl) | - | - | 0,50 | - |
| Octyldodecanol | - | 2,00 | - | - |
| Isopropyl Palmitate | 1,50 | - | - | - |
| Dimethicon | 0,50 | 1,00 | 1,00 | - |
| Glycerin | 3,00 | 5,00 | 7,00 | 9,00 |
| Methylparaben | 0,20 | 0,15 | - | - |
| Phenoxyethanol | 0,40 | 0,60 | 0,50 | 0,50 |
| Propylparaben | 0,10 | - | - | - |
| Methylisothiazolinon | - | - | 0,05 | - |
| Piroctone Olamine | - | - | - | 0,15 |
| Glyceryl Caprylat | - | - | - | 0,20 |
| Carbomer | 0,20 | - | 0,15 | 0,15 |
| Natrium Polyacrylat | - | 0,40 | - | - |
| Xanthan Gummi | 0,10 | - | 0,10 | - |
| Acrylates/C10-30 Alkyl Acrylate Crosspolymer | - | 0,10 | - | 0,10 |
| Tapioka Stärke | 0,50 | - | 0,50 | - |
| Nylon-12 (1,8-Diazacyclotetradecan-2,7-dion Homopolymer) | 1,00 | - | - | 1,00 |
| Polymethylsilsesquioxane | - | 1,00 | 1,00 | - |
| Aluminum Stärke Octenylsuccinat | - | 1,00 | - | 1,00 |
| N-(4-(2,4-Dihydroxyphenyl)thiazol-2-yl)-isobutyramid | 0.25 | 0.15 | 0.30 | 0.35 |
| N-(4-(2,4-Dihydroxyphenyl)thiazol-2-yl)-pivalamid | 0.10 | 0.10 | 0.15 | 0.20 |
| N-(4-(2,4-Dihydroxyphenyl)thiazol-2-yl)-butyramid | 0.01 | 0.25 | 0.15 | 0.10 |
| N-(4-(2,4-Dihydroxyphenyl)thiazol-2-yl)-cyclohexancarboxamid | 0,20 | 0.10 | 0.05 | 0.30 |
| Glycyrrhiza Inflata Root Extrakt | 0,03 | 0,05 | 0,05 | 0,03 |
| Titandioxid | - | 1,00 | - | - |
| Octocrylene | 1,00 | 2,00 | 1,00 | 1,00 |
| Bis-Ethylhexyloxyphenol Methoxyphenyl Triazin | 1,00 | 1,00 | 2,00 | 2,00 |
| 2-Ethylhexyl Methoxycinnamat | 2,00 | 2,00 | 1,00 | 2,00 |
| Homosalat (3,3,5-Trimethylcyclohexylsalicylat) | 1,00 | 1,00 | 2,00 | 1,00 |
| Benzophenon-3 | 0,50 | 1,00 | 0,50 | 1,00 |
| 4-Methylbenzylidencampher | 1,00 | 0,50 | 1,00 | 0,50 |
| Natriumhydroxid | q.s. | q.s. | q.s. | q.s. |
| Trinatrium EDTA | 0,15 | - | 0,15 | - |
| 1-(1,2,3,4,5,6,7,8-octahydro-2,3,8,8,-tetramethyl-2-naphthyl)ethan-1-one | 0,1 | - | q.s. | q.s. |
| Geraniol | - | 0,05 | - | - |
| Hexylcinnamal | - | - | 0,05 | - |
| Parfüm | 0,10 | 0,20 | 0,30 | 0,20 |
| Wasser | ad 100 | ad 100 | ad 100 | ad 100 |

| **Rezepturbeispiele** | **9** | **10** | **11** | **12** |
|---|---|---|---|---|
| **Chemische Bezeichnung** | **Gew.-%** | **Gew.-%** | **Gew.-%** | **Gew.-%** |
| Polyglyceryl-3 Methylglucose Distearat | 2,00 | 2,50 | 2,50 | 2,50 |
| Sorbitan Stearate | 1,50 | 3,00 | 1,50 | 3,00 |
| C12-15 Alkyl Benzoat | 2,50 | 2,50 | 2,50 | 2,50 |
| Caprylsäure/Caprinsäure Triglyceride | 2,50 | 2,50 | 2,50 | 2,50 |
| Stearyl Alkohol | 1,00 | 1,50 | 1,00 | 1,50 |
| Cyclomethicon | 3,00 | 1,00 | 2,00 | 1,00 |
| Isopropyl Myristat | - | 2,50 | 2,00 | 2,50 |
| Isopropyl Palmitat | 2,00 | - | 1,00 | - |
| EThylhexyl Stearat | - | 1,50 | - | - |
| Dimethicon | - | 1,00 | - | 1,00 |
| Decyl Oleate | - | - | 1,50 | - |
| Glycerin | 5,00 | 7,50 | 3,00 | 7,50 |
| Butyrospermum Parkii Butter | 2,00 | - | - | - |
| Squalane | 0,50 | - | - | - |
| Methylparaben | 0,20 | 0,20 | - | 0,10 |
| Phenoxyethanol | 0,40 | 0,40 | 0,40 | 0,40 |
| Propylparaben | 0,10 | - | - | - |
| Benzethonium chlorid | - | - | 0,10 | - |
| Caprylyl Glykol | - | 0,20 | - | - |
| Ethylhexylglycerin | - | 0,20 | - | 0,2 |
| Carbomer | 0,15 | 0,10 | 0,15 | 0,10 |
| Ammonium Acryloyldimethyltaurate/VP Copolymer | - | 0,20 | - | 0,20 |
| Carrageenan | 0,10 | - | 0,15 | - |
| Trinatrium EDTA | - | 1,00 | - | 1,00 |
| Tapioka Stärke | - | 1,00 | 1,00 | - |
| Distärke Phosphat | - | 1,00 | - | 1,00 |
| Acrylonitrile-methacrylonitrile-methylmethacrylate Copolymer + Isopentane + Magnesium Hydroxide | - | - | 1,00 | 1,00 |
| N-(4-(2,4-Dihydroxyphenyl)thiazol-2-yl)-isobutyramid | 0.01 | 0.25 | 0.15 | 0.10 |
| N-(4-(2,4-Dihydroxyphenyl)thiazol-2-yl)-pivalamid | 0,20 | 0.10 | 0.05 | 0.30 |
| N-(4-(2,4-Dihydroxyphenyl)thiazol-2-yl)-butyramid | 0.25 | 0.15 | 0.30 | 0.35 |
| N-(4-(2,4-Dihydroxyphenyl)thiazol-2-yl)-cyclohexancarboxamid | 0.10 | 0.10 | 0.15 | 0.20 |
| Diethylamino Hydroxybenzoyl Hexyl Benzoate | 1,00 | 2,00 | 1,00 | 1,00 |
| Ethylhexyl Methoxycinnamat | 1,00 | 1,00 | 2,00 | 2,00 |
| Butyl Methoxydibenzoylmethan | 2,00 | 2,00 | 1,00 | 2,00 |
| Octocrylen | 1,00 | 1,00 | 2,00 | 1,00 |
| 2,4-Bis-[5-](dimethylpropyl)benzoxazol-2-yl-(4-phenyl)-imino]-6-(2-ethylhexyl)-imino-1,3,5-triazin | 0,50 | 1,00 | 0,50 | 1,00 |
| Methylen-bis-benzotriazolyltetramethylbutylphenol | 1,00 | 0,50 | 1,00 | 0,50 |
| Polysilicone-15 | 0,50 | 0,50 | 0,50 | 0,50 |
| Benzol-1,4-di(2-oxo-3-bornyliden-methyl-10-sulfonsäure | 0,50 | 0,50 | 0,50 | 0,50 |
| Titandioxid | - | - | 1,00 | - |
| Natriumhydroxid | q.s. | q.s. | q.s. | q.s. |
| Ubiquinone | 0,10 | - | - | - |
| Natrium Metabisulfit | - | 0,15 | - | - |
| BHT (tert-Butylhydroxytoluol) | - | - | 0,05 | - |
| Linalyacetat | 0,05 | - | - | - |
| Hexylsalicylat | - | 0,05 | - | - |
| Benzylsalicylat | - | - | 0,01 | - |
| Parfüm | q.s. | q.s. | q.s. | q.s. |
| Wasser | ad 100 | ad 100 | ad 100 | ad 100 |

| **Rezepturbeispiele** | **13** | **14** | **15** | **16** |
|---|---|---|---|---|
| **Chemische Bezeichnung** | **Gew.-%** | **Gew.-%** | **Gew.-%** | **Gew.-%** |
| PEG-40 Stearate | 0,80 | 1,00 | 1,00 | 1,00 |
| Glyceryl Stearate | 2,50 | 3,00 | 3,00 | 3,00 |
| C12-15 Alkyl Benzoate | 2,00 | 2,50 | 2,00 | 2,00 |
| Caprylsäure/Caprinsäure Triglycerid | 2,00 | 2,50 | 2,50 | 2,00 |
| Cetylstearylalkohol | 3,00 | 3,00 | 3,00 | 3,00 |
| Cyclomethicon | 2,00 | 2,00 | 2,00 | 2,00 |
| Dicaprylyl Carbonat | - | 2,00 | 2,50 | 2,50 |
| Octyldodecanol | 1,00 | - | - | 1,50 |
| Trüsostearin | - | 0,50 | - | 1,00 |
| Butyrospermum Parkii Butter | 2,00 | - | - | - |
| Octyldodecyl Myristat | 1,00 | - | 1,50 | 1,00 |
| Dimethicon | 1,00 | 1,00 | 1,00 | 1,00 |
| Glycerin | 7,50 | 5,00 | 9,0 | 7,50 |
| Methylparaben | 0,20 | - | 0,10 | - |
| Phenoxyethanol | 0,40 | 0,50 | 0,40 | 0,40 |
| Propylparaben | 0,10 | - | - | - |
| Glyceryl Caprylat | - | 0,25 | - | - |
| Pentylenglykol | - | 0,50 | - | - |
| Butylenglykol | - | - | 3,00 | - |
| Carbomer | 0,15 | 0,10 | 0,10 | 0,15 |
| Natrium Polyacrylat | - | 0,20 | 0,20 | - |
| Xanthan Gummi | 0,10 | - | - | - |
| Acrylates/C10-30 Alkyl Acrylate Crosspolymer | - | - | - | 0,1 |
| Trinatrium EDTA + Wasser (20%ige wässrige Lösung) | - | 1,00 | 1,00 | 1,00 |
| Tapioca Stärke | - | 1,00 | 1,00 | 1,00 |
| Distärke Phosphat | - | 1,00 | 1,00 | 1,00 |
| Aluminum Stärke Octenylsuccinat | 2,00 | - | - | - |
| Acrylonitrile-methacrylonitrile-methylmethacrylate Copolymer + Isopentane + Magnesium Hydroxide | 1,00 | - | - | - |
| N-(4-(2,4-Dihydroxyphenyl)thiazol-2-yl)-isobutyramid | 0,10 | 0,15 | 0,10 | 0,01 |
| Ethylhexyl Methoxycinnamat | 1,00 | 2,00 | 1,00 | 1,00 |
| Diethylamino Hydroxybenzoyl Hexyl Benzoat | 0,50 | 1,00 | 2,00 | 1,00 |
| Homosalate (3,3,5-Trimethylcyclohexylsalicylat) | 2,00 | 2,00 | 1,00 | 2,00 |
| Phenylbenzimidazolsulfonsäure | 1,00 | 1,00 | 2,00 | 1,00 |
| Dinatriumphenyldibenzimidazoltetrasulfonat | 0,50 | 1,00 | 0,50 | 1,00 |
| Diethylhexyl-Butamidotriazone | 1,00 | 0,50 | 1,00 | 0,50 |
| Drometrizol trisiloxan | 0,50 | 1,00 | 1,00 | 0,50 |
| Isoamyl p-Methoxycinnamate | 0,50 | 0,50 | 0,50 | 0,50 |
| Titandioxid | - | - | 1,00 | - |
| Glyceryl Glucoside | 3,00 | - | - | - |
| Kurzkettige Hyaluronsäure | - | 0,10 | - | - |
| Langkettige Hyaluronsäure | - | - | 0,10 | - |
| 4-Butyl-Resorcin | - | - | - | 0,30 |
| Magnolia-Rinden-Extrakt | 0,10 | - | - | - |
| Octadecen Dioic Säure | - | 0,05 | - | - |
| Folsäure | - | - | 0,01 | - |
| Carnitin | - | - | - | 0,50 |
| Kreatin | 0,10 | - | - | - |
| Alpha-Glucosylrutin | - | 0,01 | - | - |
| Taurin | - | - | 0,10 | - |
| Maulbeeren-Wurzel-Extrakt | - | - | - | 0,20 |
| Natrium Metabisulfit | 0,10 | - | - | - |
| Diethylhexyl Syringylidenmalonat | 0,13 | 0,13 | - | - |
| Natriumhydroxid | q.s. | q.s. | q.s. | q.s. |
| 3-Methyl-5-phenyl-1-pentanol | 0,10 | - | - | - |
| Coumarin | - | 0,05 | - | - |
| Ethyllinalool | - | - | 0,10 | - |
| Ascorbylpalmitat | 0,10 | - | - | - |
| Parfüm | q.s. | q.s. | q.s. | q.s. |
| Wasser | ad 100 | ad 100 | ad 100 | ad 100 |

| **Rezepturbeispiele** | **17** | **18** | **19** | **20** |
|---|---|---|---|---|
| **Chemische Bezeichnung** | **Gew.-%** | **Gew.-%** | **Gew.-%** | **Gew.-%** |
| Glyceryl Stearate Citrat | 2,00 | 2,00 | 2,00 | 2,00 |
| Isopropyl Palmitat | 3,00 | 2,00 | 3,00 | 1,00 |
| Cetylstearylalkohol | 4,00 | 3,00 | 3,00 | - |
| Cetylalkohol | - | - | - | 4,00 |
| Caprylsäure/Caprinsäure Triglycerid | 3,00 | 2,50 | 2,00 | 3,00 |
| C12-15 Alkyl Benzoat | 3,00 | 2,50 | 2,00 | 2,00 |
| Cyclomethicon | 1,00 | - | 1,00 | - |
| Dicaprylyl Carbonat | - | - | 2,50 | - |
| Dimethicon | - | 0,50 | - | - |
| Octyldodecyl Myristat | - | 1,00 | - | - |
| Glycerin | 4,00 | 6,00 | 5,00 | 6,00 |
| Methylparaben | 0,20 | - | 0,10 | - |
| Phenoxyethanol | 0,40 | 0,40 | 0,40 | 0,40 |
| Piroctone Olamine | - | - | - | 0,10 |
| Ethylhexylglycerin | - | 0,30 | - | - |
| Glyceryl Caprylate | - | 0,30 | - | - |
| 2-Methyl-1,3-propandiol | - | 2,00 | - | 2,00 |
| Carbomer | 0,20 | 0,10 | 0,15 | - |
| Natrium Polyacrylate | - | 0,40 | - | - |
| Xanthan Gummi | 0,10 | - | - | 0,15 |
| Acrylates/C10-30 Alkyl Acrylate Crosspolymer | - | - | 0,10 | 0,20 |
| Acrylonitrile-methacrylonitrile-methylmethacrylate Copolymer + Isopentane + Magnesium Hydroxide | 0,50 | - | 0,50 | - |
| Aluminum Stärke Octenylsuccinat | - | 1,00 | - | 1,00 |
| Methyl Methacrylate Crosspolymer | 1,00 | | - | 1,00 |
| Glycyrrhiza Inflata Wurzel Extrakt | 0,03 | - | - | - |
| Vitamin C/Ascorbinsäure | - | 3,00 | - | - |
| Glycin Soja Keim Extrakt | - | - | 0,50 | - |
| Arctium Lappa Wurzel Extrakt | - | - | - | 0,30 |
| Pimpinella Anisum Frucht Extrakt | 4,00 | - | - | - |
| Glycyrrhitinsäure | - | 0,10 | - | - |
| N-Acetylhydroxyprolin | - | - | 0,10 | - |
| Niacinamid | - | - | - | 0,20 |
| Magnesium-Ascorbylphosphat | 0,10 | - | - | - |
| Ellagsäure | - | 0,01 | - | - |
| Süßholzwurzel-Extrakt | - | - | 0,10 | - |
| Meersalz | - | - | - | 0,05 |
| Isoserinol | 1,00 | - | - | - |
| Dihydroxypropyltrimonium chloride | - | 0,80 | - | - |
| N-(4-(2,4-Dihydroxyphenyl)thiazol-2-yl)-isobutyramid | 0,25 | 0,30 | 0,01 | 0,05 |
| Titandioxid | - | 1,00 | - | 1,00 |
| Bis-Ethylhexyloxyphenol Methoxyphenyl Triazin | 1,00 | 2,00 | 1,00 | 1,00 |
| Octocrylen | 1,00 | 1,00 | 2,00 | 2,00 |
| Butyl Methoxydibenzoylmethan | 2,00 | 2,00 | 1,00 | 2,00 |
| Ethylhexyl Salicylat | 1,00 | 1,00 | 2,00 | 1,00 |
| Ethylhexyl Triazon | 0,50 | 1,00 | 0,50 | 2,00 |
| Benzophenon-4 | 1,00 | 0,50 | 1,00 | 0,50 |
| Dinatriumphenyldibenzimidazoltetrasulfonat | 1,00 | 2,00 | 0,50 | 0,50 |
| 4-Methylbenzylidencampher | 0,50 | 0,50 | 0,50 | 0,50 |
| Citronellol | 0,05 | - | 0,05 | - |
| Coumarin | 0,05 | 0,05 | - | 0,05 |
| Triethylcitrat | - | - | 0,05 | 0,05 |
| Natriumhydroxid | q.s. | q.s. | q.s. | q.s. |
| Wasser | ad 100 | ad 100 | ad 100 | ad 100 |

| **Rezepturbeispiele** | **21** | **22** | **23** | **24** |
|---|---|---|---|---|
| **Chemische Bezeichnung** | **Gew.-%** | **Gew.-%** | **Gew.-%** | **Gew.-%** |
| Sucrose Polystearate + Hydrogynated Polyisobutene | 1,00 | 1,00 | 2,00 | 2,00 |
| Natrium Stearoyl Glutamat | 0,20 | 0,20 | 0,30 | 0,30 |
| C12-15 Alkyl Benzoat | 1,50 | 1,50 | - | - |
| Cetyl Alkohol | 0,50 | 0,50 | - | - |
| Cyclomethicon | 10,00 | 10,00 | 5,00 | 5,00 |
| Dimethicon | 3,00 | 3,00 | 2,50 | 2,50 |
| Glycerin | 7,50 | 7,50 | 5,00 | 5,00 |
| Isopropyl Stearat | 1,00 | 1,00 | 2,00 | 2,00 |
| Paraffinum Liquidum (Mineralöl) | 3,00 | 3,00 | 1,00 | 1,00 |
| Methylparaben | 0,10 | - | - | 0,10 |
| Ethylhexylglycerin | - | - | 0,30 | 0,10 |
| Propylparaben | 0,10 | - | - | - |
| Methylisothiazolinon | - | 0,05 | - | - |
| Phenoxyethanol | 0,40 | 0,50 | 0,40 | 0,40 |
| Ascorbylglucosid | 0,10 | - | - | - |
| Undecenoylphenylalanin | - | 0,50 | - | - |
| Kojisäure | - | - | 0,10 | - |
| Arbutin | - | - | - | 0,01 |
| Betain | 0,20 | - | - | - |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,10 | 0,25 | 0,15 | 0,10 |
| N-(4-(2,4-Dihydroxyphenyl)thiazol-2-yl)-butyramid | 0,15 | 0,15 | 0,01 | 0,06 |
| Ethylhexyl Methoxycinnamat | 1,00 | 2,00 | 1,00 | 1,00 |
| Butyl Methoxydibenzoylmethan | 1,00 | 1,00 | 2,00 | 2,00 |
| Phenylbenzimidazolsulfonsäure | 2,00 | 2,00 | 1,00 | 2,00 |
| Polysilicone-15 | 1,00 | 1,00 | 2,00 | 1,00 |
| Diethylhexyl-Butamidotriazone | 0,50 | 1,00 | 0,50 | 1,00 |
| (2-{4-[2-(4-Diethylamino-2-hydroxybenzoyl)-benzoyl]-piperazin-1-carbonyl}-phenyl)-(4-diethylamino-2-hydroxyphenyl)-methanon | 1,00 | 0,50 | 1,00 | 0,50 |
| Acrylates/Octylacrylamid Copolymer | - | 1,00 | - | - |
| Butylenglykol | - | - | 3,00 | - |
| Polymethylsilsesquioxan | - | - | 1,00 | 1,00 |
| Prunus Amygdalus Dulcis Oil | - | - | 1,00 | - |
| Nylon-12 (1,8-Diazacyclotetradecan-2,7-dion Homopolymer) | - | 1,00 | 1,00 | - |
| Distärke Phosphat | - | 1,00 | - | 1,00 |
| Methyl Methacrylat Crosspolymer | 1,00 | - | - | - |
| Aluminum Starch Octenylsuccinat | 1,00 | - | - | - |
| Ammonium Acryloyldimethyltaurate/VP Copolymer | - | - | 0,25 | 0,25 |
| Xanthan Gummi | 0,10 | - | - | 0,10 |
| Acrylates/C10-30 Alkyl Acrylate Crosspolymer | 0,25 | 0,10 | - | - |
| Carbomer | - | 0,10 | 0,10 | - |
| Hexylcinnamal | 0,05 | 0,10 | - | 0,10 |
| 1-(1,2,3,4,5,6,7,8-octahydro-2,3,8,8,-tetramethyl-2-naphthyl)ethan-1-one | - | 0,10 | 0,10 | - |
| Linalool | - | - | 0,05 | 0,05 |
| Parfüm | 0,20 | 0,20 | 0,20 | 0,20 |
| Natriumhydroxid | q.s. | q.s. | q.s. | q.s. |
| Wasser | ad 100 | ad 100 | ad 100 | ad 100 |

| **Rezepturbeispiele** | **25** | **26** | **27** | **28** |
|---|---|---|---|---|
| **Chemische Bezeichnung** | **Gew.-%** | **Gew.-%** | **Gew.-%** | **Gew.-%** |
| Natrium Cetearyl Sulfat | 0,15 | 0,15 | - | 0,15 |
| Glyceryl Stearate SE | 2,00 | 2,00 | - | 1,50 |
| Natrium Stearoyl Glutamate | - | - | 0,30 | |
| C12-15 Alkyl Benzoat | 2,50 | 2,50 | 2,50 | 2,50 |
| Octyldodecanol | 1,00 | 1,00 | - | - |
| Caprylsäure/Caprinsäure Triglycerid | 2,00 | 2,00 | 2,00 | 2,00 |
| Cetylstearylalkohol | 2,00 | 2,00 | 3,00 | 1,00 |
| Cyclomethicon | 1,50 | 1,50 | 2,50 | 2,50 |
| Glyceryl Stearate | - | - | 2,00 | - |
| Dimethicon | 0,50 | 0,50 | 0,50 | 0,50 |
| Glycerin | 5,00 | 5,00 | 7,50 | 7,50 |
| Cetearyl Alkohol | 1,00 | 1,50 | 1,00 | 1,00 |
| Isopropyl Stearat | 3,00 | 3,00 | 2,00 | 2,00 |
| Paraffinum Liquidum (Mineralöl) | 2,00 | 2,00 | 1,00 | 1,00 |
| Methylisothiazolinon | - | - | - | 0,05 |
| Phenoxyethanol | 0,40 | 0,50 | 0,40 | 0,30 |
| Methylparaben | 0,15 | - | - | - |
| Propylparaben | 0,10 | - | - | - |
| Piroctone Olamine | - | 0,15 | - | - |
| Benzethonium chlorid | - | - | 0,10 | - |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | 0,30 | 0,01 | 0,05 |
| N-(4-(2,4-Dihydroxyphenyl)thiazol-2-yl)-butyramid | 0,10 | 0,25 | 0,15 | 0,10 |
| N-(4-(2,4-Dihydroxyphenyl)thiazol-2-yl)-cyclohexancarboxamid | 0,15 | 0,15 | 0,01 | 0,06 |
| Ethylhexyl Methoxycinnamat | 1,00 | 2,00 | 1,00 | 1,00 |
| Butyl Methoxydibenzoylmethan | 1,00 | 1,00 | 2,00 | 2,00 |
| Drometrizol trisiloxan | 2,00 | 2,00 | 1,00 | 1,00 |
| Benzophenon-3 | 1,00 | 1,00 | 2,00 | 1,00 |
| Benzol-1,4-di(2-oxo-3-bornylidenmethyl-10-sulfonsäure | 0,50 | 1,00 | 0,50 | 2,00 |
| Isoamyl p-Methoxycinnamat | 2,00 | 0,50 | 0,50 | 0,50 |
| Methylen-bis-benzotriazolyltetramethylbutylphenol | 1,00 | 2,00 | 0,50 | 0,50 |
| 2,4-Bis-[5-](dimethylpropyl)benzoxazol-2-yl-(4-phenyl)-imino]-6-(2-ethylhexyl)-imino-1,3,5-triazin | 0,50 | 0,50 | 0,50 | 2,00 |
| Pentylenglykol | - | 1,00 | 1,00 | - |
| ButylenGlykol | 1,00 | 1,50 | 3,00 | 3,00 |
| DipropylenGlykol | 0,50 | 1,00 | 0,80 | 0,10 |
| 2-Methyl-1,3-propandiol | - | - | - | - |
| 1,2-Hexandiol | - | - | - | 1,00 |
| Nylon-12 (1,8-Diazacyclotetradecan-2,7-dion Homopolymer) | 1,00 | 1,00 | 1,00 | 1,00 |
| Carbomer | - | - | 0,10 | 0,15 |
| Ammonium Acryloyldimethyltaurat/VP Copolymer | 0,20 | - | - | - |
| Chondrus Crispus | 0,10 | 0,10 | - | - |
| Xanthan Gummi | - | - | 0,10 | - |
| Acrylates/C10-30 Alkyl Acrylate Crosspolymer | - | 0,20 | 0,10 | 0,10 |
| Coumarin | 0,10 | - | 0,05 | 0,05 |
| Hydroxyisohexyl 3-Cyclohexencarboxaldehyde | 0,05 | 0,05 | 0,05 | 0,10 |
| 1-(1,2,3,4,5,6,7,8-octahydro-2,3,8,8,-tetramethyl-2-naphthyl)ethan-1-one | - | 0,05 | 0,10 | - |
| Parfüm | 0,20 | 0,30 | 0,40 | 0,20 |
| Natriumhydroxid | q.s. | q.s. | q.s. | q.s. |
| Wasser | ad 100 | ad 100 | ad 100 | ad 100 |

| **Rezepturbeispiele** | **29** | **30** | **31** | **32** |
|---|---|---|---|---|
| **Chemische/INCI-Bezeichnung** | **Gew.-%** | **Gew.-%** | **Gew.-%** | **Gew.-%** |
| Natrium Cetearyl Sulfat | 0,15 | 0,15 | 0,20 | 0,20 |
| Glyceryl Stearate, selbstemulgierend | 2,00 | 2,00 | 1,50 | 1,50 |
| C12-15 Alkyl Benzoat | 2,00 | 2,00 | 2,00 | 2,00 |
| Octyldodecanol | 1,00 | 1,00 | - | - |
| Caprylsäure/Caprinsäure Triglycerid | 2,00 | 2,00 | 2,00 | 2,00 |
| Cetylstearylalkohol | 2,00 | 2,00 | 1,00 | 1,00 |
| Cyclomethicon | 1,00 | 1,00 | 2,00 | 2,00 |
| Dimethicon | 0,50 | 0,50 | 1,00 | 1,00 |
| Glycerin | 5,00 | 5,00 | 7,50 | 7,50 |
| Isopropyl Palmitat | 2,50 | 2,50 | 2,00 | 2,00 |
| DMDM Hydantoin | 0,05 | 0,05 | 0,05 | 0,05 |
| Phenoxyethanol | 0,35 | 0,25 | 0,30 | 0,30 |
| Ethanol | - | - | 3,00 | 2,00 |
| Pentylenglykol | 1,00 | - | 1,00 | 1,50 |
| Zingeron | 0,10 | - | - | - |
| Dihydromyricetin | - | 0,03 | - | - |
| Weisser Tee Extrakt | - | - | 1,00 | - |
| 4-Hexyl-Resorcin | - | - | - | 0,30 |
| Phenylethyl Resorcinol | 0,50 | - | - | - |
| Ubiquinone | - | 0,10 | - | - |
| Cyanomethylphenyl Menthan Carboxamid | - | - | 0,10 | - |
| Menthoxypropanediol | - | - | - | 0,10 |
| Menthan Carboxamid Ethylpyridin | 0,10 | - | - | - |
| Hydroxyethylurea | - | 0,50 | - | - |
| Urea | - | - | 1,00 | - |
| N-(4-(2,4-Dihydroxyphenyl)thiazol-2-yl)-isobutyramid | 0,10 | 0,10 | 0,05 | 0,05 |
| Carbomer | 0,20 | 0,20 | 0,20 | 0,20 |
| Carrageenan | 0,10 | 0,10 | - | - |
| Xanthan Gummi | - | - | 0,20 | 0,20 |
| Acrylates/C10-30 Alkyl Acrylate Crosspolymer | - | - | - | 0,15 |
| Natrium Polyacrylat | - | 0,20 | - | - |
| Diethylhexyl 2,6-Naphthalat | - | - | 1,00 | - |
| Phenylbenzimidazolsulfonsäure | 1,00 | 2,00 | 1,00 | 1,00 |
| Titandioxid | 1,00 | 1,00 | 2,00 | 2,00 |
| Diethylamino Hydroxybenzoyl Hexyl Benzoat | 2,00 | 2,00 | 1,00 | 2,00 |
| Octocrylene | 1,00 | 1,00 | 2,00 | 1,00 |
| Ethylhexylsalicylat | 0,50 | 1,00 | 0,50 | 1,00 |
| Benzophenon-4 | 1,00 | 0,50 | 1,00 | 0,50 |
| Isoamyl p-Methoxycinnamat | 2,00 | 0,50 | 0,50 | 2,00 |
| 3,3,5-Trimethylcyclohexylsalicylat | | 1,00 | - | - |
| Distärkepjosphat | - | 1,00 | 1,00 | - |
| Methyl Methacrylate Crosspolymer | 1,00 | - | - | 1,00 |
| Polymethylsilsesquioxane | - | - | 1,00 | 1,00 |
| Acrylonitrile-methacrylonitrile-methylmethacrylate Copolymer + Isopentane + Magnesium Hydroxid | 1,00 | 1,00 | - | - |
| 1-(1,2,3,4,5,6,7,8-octahydro-2,3,8,8,-tetramethyl-2-naphthyl)ethan-1-one | - | 0,10 | 0,10 | 0,05 |
| Hydroxyisohexyl 3-Cyclohexencarboxaldehyde | 0,05 | 0,05 | 0,10 | - |
| Linalylacetat | 0,10 | - | 0,05 | 0,05 |
| Parfüm | 0,15 | 0,15 | 0,30 | 0,30 |
| Natriumhydroxid | q.s. | q.s. | q.s. | q.s. |
| Wasser | ad 100 | ad 100 | ad 100 | ad 100 |

| **Rezepturbeispiele** | **33** | **34** | **35** | **36** |
|---|---|---|---|---|
| **Chemische/INCI-Bezeichnung** | **Gew.-%** | **Gew.-%** | **Gew.-%** | **Gew.-%** |
| Natrium Cetearyl Sulfat | 0,15 | 0,15 | 0,15 | 0,15 |
| Glyceryl Stearate, selbstemulgierend | 1,00 | 1,00 | 1,00 | 1,00 |
| C12-15 Alkyl Benzoat | 2,00 | 2,50 | 2,00 | 2,00 |
| Isopropyl Palmitat | 3,50 | 3,00 | 2,50 | 3,50 |
| Dimethicon | 1,00 | 1,00 | 1,00 | 1,0 |
| Cetylstearylalkohol | 1,00 | 1,00 | 1,00 | 1,00 |
| Octyldodecyl Myristate | - | - | - | 1,00 |
| Butyrospermum Parkii Butter | - | - | 1,00 | - |
| Glycerin | 7,00 | 3,00 | 9,00 | 5,00 |
| Carbomer | 0,10 | 0,15 | 0,10 | 0,10 |
| Acrylates/C10-30 Alkyl Acrylate Crosspolymer | 0,15 | 0,10 | 0,10 | 0,15 |
| Xanthan Gummi | 0,15 | 0,15 | 0,15 | 0,15 |
| Phenylbenzimidazolsulfonsäure | 1,00 | 1,00 | 0,50 | 1,00 |
| Butyl Methoxydibenzoylmethan | 1,50 | 1,50 | 1,50 | 1,50 |
| Ethylhexyl Salicylat | 2,00 | 2,50 | 2,50 | 2,50 |
| Octocrylene | 1,50 | 1,50 | 2,50 | 1,50 |
| Bis-Ethylhexyloxyphenolmethoxyphenyltriazin | 1,00 | 2,00 | 1,50 | 2,00 |
| Drometrizol trisiloxan | 2,00 | 1,50 | 1,00 | 0,50 |
| Titanium Dioxide + Trimethoxycaprylylsilan | 1,00 | - | 1,00 | - |
| Aluminum Stärke Octenylsuccinat | - | 1,00 | - | 0,50 |
| Methyl Methacrylate Crosspolymer | 0,50 | - | 0,50 | - |
| Nylon-12 (1,8-Diazacyclotetradecan-2,7-dion Homopolymer) | 0,50 | - | 1,00 | - |
| Tapioka Stärke | 0,50 | 0,50 | - | 1,00 |
| Phenoxyethanol | 0,50 | 0,50 | 0,50 | 0,40 |
| Ethylhexylglycerin | 0,25 | - | 0,25 | - |
| 1,2-Hexandiol | - | 1,00 | - | 3,00 |
| Caprylyl Glykol | - | 0,30 | 0,30 | - |
| 2-Methyl-1,2-propandiol | 2,00 | 2,00 | 2,00 | - |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | 0,30 | 0,01 | 0,05 |
| Ethyllinalool | 0,05 | - | 0,05 | - |
| 3-Methyl-5-phenyl-1-pentanol | - | 0,05 | - | 0,05 |
| Geraniol | 0,05 | - | 0,05 | - |
| Natriumhydroxid | q.s. | q.s. | q.s. | q.s. |
| Wasser | ad 100 | ad 100 | ad 100 | ad 100 |

| **Rezepturbeispiele** | **37** | **38** | **39** | **40** |
|---|---|---|---|---|
| **Chemische/INCI-Bezeichnung** | **Gew.-%** | **Gew.-%** | **Gew.-%** | **Gew.-%** |
| Polyglycerly-10 Stearat | 0,20 | 0,20 | 0,20 | 0,20 |
| Glyceryl Stearat | 3,00 | 0,50 | 0,50 | 0,50 |
| C12-15 Alkyl Benzoat | 4,00 | 2,00 | 1,50 | 2,50 |
| Isopropyl Palmitat | 4,00 | 1,00 | 2,00 | 2,50 |
| Caprylsäure/Caprinsäure Triglycerid | 4,00 | 3,00 | 2,00 | 2,50 |
| Hydrogenated Coco-Glycerides | 3,00 | - | - | 2,00 |
| Butyrospermum Parkii Butter | 3,00 | - | 2,50 | - |
| Cetylstearylalkohol | 5,00 | 3,50 | 4,00 | 3,00 |
| Paraffinum Liquidum (Mineralöl) | - | - | - | 1,00 |
| Glycerin | 5,00 | 3,00 | 7,00 | 9,00 |
| Acrylates/C10-30 Alkyl Acrylate Crosspolymer | 0,30 | 0,20 | 0,15 | 0,20 |
| Methylisothiazolinon | 0,05 | - | - | 0,05 |
| Phenoxyethanol | 0,50 | 0,40 | 0,40 | 0,40 |
| Carbomer | 0,10 | 0,15 | 0,10 | 0,10 |
| Methylparaben | - | 0,10 | 0,10 | - |
| Propylparaben | - | 0,10 | - | - |
| Nylon-12 (1,8-Diazacyclotetradecan-2,7-dion Homopolymer) | 1,00 | 0,50 | - | - |
| Polymethylsilsesquioxane | - | 1,00 | 0,50 | - |
| Methyl Methacrylate Crosspolymer | - | - | 1,00 | 0,50 |
| Tapioka Stärke | 0,50 | - | - | 0,50 |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | 0,30 | 0,01 | 0,05 |
| N-(4-(2,4-Dihydroxyphenyl)thiazol-2-yl)-butyramid | 0,10 | 0,25 | 0,15 | 0,10 |
| N-(4-(2,4-Dihydroxyphenyl)thiazol-2-yl)-cyclohexancarboxamid | 0,01 | 0,60 | 1,00 | 0,20 |
| Benzophenon-4 | 1,00 | 2,00 | 1,50 | 0,50 |
| Ethylhexyl Triazone | 2,00 | 0,50 | 1,00 | 2,00 |
| Isoamyl p-Methoxycinnamat | 1,00 | 0,50 | 2,00 | 1,50 |
| Polysilicone-15 | 1,50 | 1,00 | 1,00 | 0,50 |
| Benzophenon-3 | 0,30 | 0,80 | 1,00 | 0,55 |
| Ethanol | 3,00 | - | 2,00 | - |
| Geraniol | 0,05 | 0,05 | - | - |
| Benzylsalicylat | - | 0,05 | 0,05 | - |
| Ethyllinalool | - | - | 0,05 | 0,05 |
| Parfüm | 0,20 | 0,15 | 0,30 | 0,30 |
| Natriumhydroxid | q.s. | q.s. | q.s. | q.s. |
| Wasser | ad 100 | ad 100 | ad 100 | ad 100 |

| **Rezepturbeispiele** | **41** | **42** | **43** | **44** |
|---|---|---|---|---|
| **Chemische/INCI-Bezeichnung** | **Gew.-%** | **Gew.-%** | **Gew.-%** | **Gew.-%** |
| Polyglycerly-10 Stearat | 0,20 | 0,20 | 0,15 | 0,15 |
| C12-15 Alkyl Benzoat | 2,50 | 2,50 | 2,00 | 3,00 |
| Isopropyl Palmitat | 2,50 | 2,50 | 2,00 | 2,00 |
| Caprylsäure/Caprinsäure Triglycerid | 2,00 | 2,50 | 1,00 | 2,00 |
| Glyceryl Stearat | 1,00 | 1,00 | 0,50 | 0,50 |
| Octyldodecanol | 0,50 | - | - | 1,00 |
| Cyclomethicon | - | - | 0,50 | 0,50 |
| Butyl Methoxydibenzoylmethan | 1,00 | 2,00 | 2,00 | 1,00 |
| Octocrylene | 0,50 | 2,00 | 3,00 | 2,00 |
| Ethylhexyl Salicylat | 2,00 | 1,00 | 1,00 | 1,50 |
| Phenylbenzimidazolsulfonsäure | 1,00 | 1,00 | 0,50 | 1,50 |
| Benzol-1,4-di(2-oxo-3-bornylidenmethyl-10-sulfonsäure | 0,50 | 1,00 | 0,50 | 2,00 |
| Isoamyl p-Methoxycinnamat | 2,00 | 0,50 | 0,50 | 0,50 |
| Methylen-bis-benzotriazolyltetramethylbutylphenol | 1,00 | 2,00 | 0,50 | 0,50 |
| 2,4-Bis-[5-](dimethylpropyl)benzoxazol-2-yl-(4-phenyl)-imino]-6-(2-ethylhexyl)-imino-1,3,5-triazin | 0,50 | 0,50 | 0,50 | 2,00 |
| Titandioxid | - | 1,00 | - | 1,00 |
| 3,3,5-Trimethylcyclohexylsalicylat | - | - | 1,00 | 1,00 |
| Glycerin | 9,00 | 5,00 | 7,00 | 7,00 |
| Tapioka Stärke | 1,00 | 1,00 | - | - |
| Acrylonitrile-methacrylonitrile-methylmethacrylate Copolymer + Isopentane + Magnesium Hydroxide | - | 1,00 | 0,50 | - |
| Aluminum Stärke Octenylsuccinat | - | - | 1,00 | 1,00 |
| Distärkephosphat | - | - | - | 1,00 |
| Methylisothiazolinon | 0,05 | 0,05 | - | - |
| Phenoxyethanol | 0,50 | 0,50 | 0,40 | 0,40 |
| Benzethoniumchlorid | - | - | 0,10 | 0,10 - |
| Ethylhexylglycerin | - | - | 0,10 | 0,10 - |
| Methylparaben | - | - | - | 0,20 |
| Carbomer | 0,25 | 0,20 | 0,20 | 0,20 |
| Acrylates/C10-30 Alkyl Acrylate Crosspolymer | 0,20 | - | - | 0,15 |
| Ammonium Acryloyldimethyltaurate/VP Copolymer | - | 0,25 | - | - |
| Natrium Polyacrylat | - | - | 0,30 | 0,30 - |
| Xanthan Gummi | - | - | - | 0,15 |
| N-(4-(2,4-Dihydroxyphenyl)thiazol-2-yl)-isobutyramid | 0,10 | 0,10 | 0,05 | 0,05 |
| Ethanol | 3,00 | 3,00 | - | - |
| Butylenglykol | - | - | 2,00 | 2,00 |
| Coumarin | - | 0,05 | 0,05 | - |
| Hexylcinnamal | 0,05 | 0,05 | - | 0,05 |
| Hexylsalicylat | - | - | 0,05 | 0,05 |
| Parfüm | 0,15 | 0,20 | 0,25 | 0,30 |
| Natriumhydroxid | q.s. | q.s. | q.s. | q.s. |
| Wasser | ad 100 | ad 100 | ad 100 | ad 100 |

| **Rezepturbeispiele** | **45** | **46** | **47** | **48** |
|---|---|---|---|---|
| **Chemische/INCI-Bezeichnung** | **Gew.-%** | **Gew.-%** | **Gew.-%** | **Gew.-%** |
| Kalium Cetylphosphat | 0,20 | 0,20 | 0,20 | 0,20 |
| Dicaprylyl Carbonat | - | 1,00 | - | - |
| C12-15 Alkyl Benzoat | 2,50 | 2,00 | 1,00 | 3,00 |
| Isopropyl Palmitat | 2,50 | 2,00 | 3,00 | 1,00 |
| Caprylsäure/Caprinsäure Triglycerid | 2,50 | 2,00 | 1,50 | 2,00 |
| Cera Microcristallina | - | - | - | 0,50 |
| Cylcomethicon | 0,25 | - | 0,50 | 0,50 |
| Diethylhexyl 2,6-Naphthalat | - | 0,50 | - | 1,00 |
| Diethylamino Hydroxybenzoyl Hexyl Benzoat | - | 1,00 | - | 1,00 |
| Ethylhexyl Salicylat | 1,00 | 0,50 | 2,00 | 1,00 |
| Octocrylen | 2,00 | 1,00 | 3,00 | 2,00 |
| Benzophenon-3 | 0,50 | 2,00 | 0,50 | 0,50 |
| Dinatriumphenyldibenzimidazoltetrasulfonat | 0,50 | 0,50 | 0,50 | 0,50 |
| 4-Methylbenzylidencampher | 0,01 | 0,20 | 0,10 | 0,05 |
| Diethylhexyl-Butamidotriazone | 1,00 | 0,50 | 0,15 | 0,60 |
| Glycerin | 5,00 | 7,00 | 9,00 | 7,00 |
| Acrylates/C10-30 Alkyl Acrylate Crosspolymer | 0,10 | 0,30 | - | 0,10 |
| Natrium Polyacrylat | 0,30 | - | - | - |
| Carbomer | - | 0,10 | 0,15 | 0,15 |
| Ammonium Acryloyldimethyltaurate/VP Copolymer | - | - | 0,25 | - |
| Chondrus Crispus Extrakt (Carrageenan) | - | - | - | 0,10 |
| Methylisothiazolinon | 0,05 | 0,05 | - | - |
| Phenoxyethanol | 0,50 | 0,50 | 0,40 | 0,40 |
| Piroctone Olamine | - | - | - | 0,20 |
| Nylon-12 (1,8-Diazacyclotetradecan-2,7-dion Homopolymer) | 0,50 | - | 0,50 | 0,50 |
| Distärkephopsphat | - | 1,00 | - | 0,50 |
| Methyl Methacrylate Crosspolymer | - | 0,50 | 0,50 | - |
| Caprylyl Glykol | - | - | 0,30 | 0,30 - |
| 1,2-Hexandiol | - | - | - | 0,50 |
| Butylenglykol | - | - | 2,00 | 2,00 |
| DMDM Hydantoin | - | - | 0,15 | 0,15 - |
| Glycyrrhiza Inflata Root Extrakt (Süßholzwurzel) | (Süßholzwurzel) | - | 0,05 | - |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | 0,30 | 0,01 | 0,05 |
| N-(4-(2,4-Dihydroxyphenyl)thiazol-2-yl)-butyramid | 0,10 | 0,25 | 0,15 | 0,10 |
| N-(4-(2,4-Dihydroxyphenyl)thiazol-2-yl)-cyclohexancarboxamid | 0,01 | 0,60 | 1,00 | 0,20 |
| Hydroxyisohexyl 3-Cyclohexencarboxaldehyd | 0,05 | 0,05 | 0,05 | - |
| Citronellol | - | 0,05 | - | 0,05 |
| Benzylsalicylat | - | - | 0,05 | 0,05 |
| Parfüm | 0,20 | 0,20 | 0,20 | 0,20 |
| Natriumhydroxid | q.s. | q.s. | q.s. | q.s. |
| Wasser | ad 100 | ad 100 | ad 100 | ad 100 |

| **Rezepturbeispiele** | **49** | **50** | **51** | **52** |
|---|---|---|---|---|
| **Chemische/INCI-Bezeichnung** | **Gew.-%** | **Gew.-%** | **Gew.-%** | **Gew.-%** |
| Kalium Cetylphosphat | 0,20 | 0,20 | 0,25 | 0,20 |
| C12-15 Alkyl Benzoat | 2,50 | 2,50 | 2,00 | 2,00 |
| Isopropyl Palmitat | 2,50 | 2,50 | - | 3,00 |
| Isopropy Stearat | - | - | 2,00 | - |
| Caprylsäure/Caprinsäure Triglycerid | 2,50 | 2,50 | 1,50 | 2,00 |
| Glyceryl Stearat | 1,00 | 1,00 | 1,25 | 1,50 |
| Octyldodecanol | - | - | 1,50 | - |
| Paraffinum Liquidum (Mineralöl) | - | - | - | 1,00 |
| Glycerin | 5,00 | 7,00 | 9,00 | 6,00 |
| Bis-Ethylhexyloxyphenol Methoxyphenyl Triazines | - | 1,00 | - | 1,00 |
| Titandioxid + Trimethoxycaprylylsilan | - | - | 1,00 | 1,00 |
| Phenylbenzimidazolsulfonsäure | 1,00 | 2,00 | 1,00 | 1,00 |
| Butyl Methoxydibenzoylmethan | 1,00 | 1,00 | 2,00 | 2,00 |
| Dinatrium Phenyl Dibenzimidazol Tetrasulfonat | 2,00 | 2,00 | 1,00 | 2,00 |
| Ethylhexyltriazon | 1,00 | 1,00 | 2,00 | 1,00 |
| Ethylhexyl Methoxycinnamat + BHT | 0,50 | 1,00 | 0,50 | 1,00 |
| 2,4-Bis-[5-](dimethylpropyl)benzoxazol-2-yl-(4-phenyl)-imino]-6-(2-ethylhexyl)-imino-1,3,5-triazin | 1,00 | 0,50 | 1,00 | 0,50 |
| Carbomer | | 0,15 | 0,20 | 0,30 |
| Acrylates/C10-30 Alkyl Acrylate Crosspolymer | 0,30 | 0,10 | 0,15 | - |
| Xanthan Gummi | | | 0,15 | 0,10 |
| Methylisothiazolinon | 0,05 | - | - | - |
| Phenoxyethanol | 0,50 | 0,50 | 0,40 | 0,40 |
| Methylparaben | - | 0,10 | - | - |
| Ethylhexylsalicylat | - | - | 0,30 | - |
| ButylenGlykol | - | - | 3,00 | 3,00 |
| Benzethonium chlorid | - | - | - | 0,10 |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | - | 0,30 | - | 0,05 |
| N-(4-(2,4-Dihydroxyphenyl)thiazol-2-yl)-butyramid | - | - | 0,15 | 0,10 |
| N-(4-(2,4-Dihydroxyphenyl)thiazol-2-yl)-cyclohexancarboxamid | - | 0,60 | 1,00 | 0,20 |
| Coumarin | - | 0,05 | - | 0,05 |
| Linalool | 0,05 | - | - | 0,05 |
| Hexylcinnamal | 0,05 | 0,05 | - | - |
| 1-(1,2,3,4,5,6,7,8-octahydro-2,3,8,8,-tetramethyl-2-naphthyl)ethan-1-on | - | - | 0,10 | - |
| Parfüm | 0,10 | 0,30 | 0,20 | 0,30 |
| BHT (tert-Butylhydroxytoluol) | 0,05 | - | - | - |
| Tocopheryl Acetat | - | 0,10 | - | - |
| Natriumhydroxid | q.s. | q.s. | q.s. | q.s. |
| Wasser | ad 100 | ad 100 | ad 100 | ad 100 |

### W/O-Emulsionen

| **Rezepturbeispiele** | **53** | **54** |
|---|---|---|
| **Chemische/INCI-Bezeichnung** | **Gew.-%** | **Gew.-%** |
| Polygylceryl-3 Diisostearat | 1,5 | 1,5 |
| PEG-40 Sorbitan Perisostearat | 2,5 | 2,5 |
| Lanolin Alkohol | 0,5 | 0,5 |
| Paraffinum Liquidum (Mineralöl) | 8 | 8 |
| Cera Microcrystallina | 2,5 | 2,5 |
| Cyclomethicon | 4 | 4 |
| Isohexadecan | 2 | 2 |
| Isopropylpalmitat | 5 | 5 |
| Iodopropynyl Butylcarbamat | - | 0,1 |
| Magnesiumsulfat | 0,5 | 0,5 |
| Kaliumsorbat | 0,1 | - |
| Benzylsalicylat | 0,1 | - |
| Drometrizol trisiloxan | 1,00 | 2,00 |
| 4-Methylbenzylidencampher | 0,20 | 2,00 |
| Homosalate | 0,50 | 1,00 |
| Benzophenon-4 | 2,00 | 0,50 |
| (2-{4-[2-(4-Diethylamino-2-hydroxy-benzoyl)-benzoyl]-piperazin-1-carbonyl}-phenyl)-(4-diethylamino-2-hydroxyphenyl)-methanon | 1,00 | 0,50 |
| N-(4-(2,4-Dihydroxyphenyl)thiazol-2-yl)-isobutyramid | 0,10 | 0,10 |
| N-(4-(2,4-Dihydroxyphenyl)thiazol-2-yl)-pivalamid | 0,25 | - |
| N-(4-(2,4-Dihydroxyphenyl)thiazol-2-yl)-butyramid | 0,10 | - |
| N-(4-(2,4-Dihydroxyphenyl)thiazol-2-yl)-cyclohexancarboxamid | 0,01 | 0,10 |
| Glycerin | 7 | 7 |
| Parfüm | q.s. | q.s. |
| Wasser | ad 100 | ad 100 |

### Deo/AT-Beispielrezepturen

| **Rezepturbeispiele** | **55** | **56** | **57** | **58** |
|---|---|---|---|---|
| **Chemische/INCI-Bezeichnung** | **Gew.- %** | **Gew.- %** | **Gew.- %** | **Gew.- %** |
| Polyethylenglykol(21)stearylether | 2,50 | 2,50 | 1,50 | 1,50 |
| Polyethylenglykol(2)stearylether | 1,50 | 1,50 | 2,50 | 2,50 |
| Polypropylenglykol(15)stearylether | 3,00 | 3,00 | 4,00 | 4,00 |
| Trinatriumsalz der Ethylendiamintetraessigsäure (20% wäßr. Lösung) | 1,50 | 1,50 | 1,50 | 1,50 |
| Persea Gratissima Öl (Avokadoöl) | 0,10 | 0,10 | 0,15 | 0,15 |
| Parfüm | q.s. | q.s. | q.s. | q.s. |
| 1-(1,2,3,4,5,6,7,8-octahydro-2,3,8,8,-tetramethyl-2-naphthyl)ethan-1-one | 0,10 | 0,05 | - | 0,05 |
| Linaylacetat | - | 0,05 | 0,05 | - |
| Citronellol | - | - | 0,05 | - |
| Triethylcitrat | - | - | - | 0,05 |
| Diethylhexyl-Butamidotriazon | 1,00 | 2,00 | 0,50 | 1,00 |
| Ethylhexylsalicylat | 2,00 | 0,50 | 0,50 | 0,50 |
| Silber Citrat | 0,10 | - | - | - |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | - | - | 0,05 |
| N-(4-(2,4-Dihydroxyphenyl)thiazol-2-yl)-butyramid | 0,10 | - | 0,15 | 0,10 |
| N-(4-(2,4-Dihydroxyphenyl)thiazol-2-yl)-cyclohexancarboxamid | 0,01 | - | - | - |
| Wasser, ad | ad 100 | ad 100 | ad 100 | ad 100 |

| **Rezepturbeispiele** | **59** | **60** | **61** | **62** |
|---|---|---|---|---|
| **Chemische/INCI-Bezeichnung** | **Gew.-%** | **Gew.-%** | **Gew.-%** | **Gew.-%** |
| Isoceteth-20 | 3,50 | 3,00 | 4,00 | 4,00 |
| Glycerylisostearat | 2,00 | 2,00 | 2,00 | 2,50 |
| Dicaprylylether | - | 0,50 | 2,00 | 2,50 |
| Capryl-Caprinsäureester | 2,00 | 1,50 | - | - |
| Aluminiumchlorhydrat | 5,00 | 5,00 | - | 3,00 |
| Persea Gratissima Öl (Avokadoöl) | - | - | 0,20 | - |
| PolyethylenGlykol(150)distearat | 0,50 | 0,50 | 1,00 | 1,00 |
| Glycerin | 4,00 | 2,00 | - | 2,00 |
| Butylenglykol | - | 3,00 | 1,00 | 2,00 |
| Propylenglykol | 3,00 | - | 3,00 | - |
| 4-[(Cyclopentylhydroxyphenylacetyl)oxy]-1,1-dimethyl-piperidiniumbromid | 0,05 | 0,10 | - | - |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | - | - | - | 0,05 |
| N-(4-(2,4-Dihydroxyphenyl)thiazol-2-yl)-butyramid | - | - | 0,15 | - |
| N-(4-(2,4-Dihydroxyphenyl)thiazol-2-yl)-cyclohexancarboxamid | - | - | 1,00 | 0,20 |
| Phenylbenzimidazolsulfonsäure | 0,50 | 1,00 | 1,00 | 2,00 |
| Isoamyl p-Methoxycinnamat | 1,00 | 0,50 | 0,50 | 0,50 |
| Geraniol | - | 0,05 | - | - |
| Ethyllinalool | - | - | 0,05 | - |
| Linalool | - | - | - | 0,10 |
| Parfüm | 0,25 | 0,50 | 0,50 | 0,75 |
| Wasser, ad | ad 100 | ad 100 | ad 100 | ad 100 |

| **Rezepturbeispiele** | **63** | **64** | **65** | **66** |
|---|---|---|---|---|
| **Chemische/INCI-Bezeichnung** | **Gew.-%** | **Gew.-%** | **Gew.-%** | **Gew.-%** |
| Polyoxyethylen(20)cetylstearylether | 3,00 | 3,00 | 4,00 | 4,00 |
| Polyoxyethylen(12)cetylstearylether | 0,50 | 0,50 | - | - |
| Glycerinstearat | 3,00 | 3,00 | 3,00 | 3,00 |
| Cetylstearylalkohol | 0,50 | 0,50 | - | - |
| Cetylpalmitat | 0,50 | 0,50 | - | - |
| Capryl-Caprinsäureester | 4,00 | 4,00 | 3,50 | 3,50 |
| Di-n-Octylether | 5,00 | 5,00 | 5,00 | 5,00 |
| PolyethylenGlykol(150)distearat | - | - | 1,00 | 1,00 |
| Glycerin | 4,00 | 4,00 | 2,00 | 2,00 |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | 0,30 | 0,01 | - |
| N-(4-(2,4-Dihydroxyphenyl)thiazol-2-yl)-butyramid | 0,10 | 0,25 | 0,15 | - |
| N-(4-(2,4-Dihydroxyphenyl)thiazol-2-yl)-cyclohexancarboxamid | 0,01 | 0,60 | 1,00 | - |
| Octocrylen | 1,00 | 1,00 | 2,00 | 2,00 |
| Benzophenon-3 | 2,00 | 2,00 | 1,00 | 2,00 |
| Hexylcinnamal | 0,05 | 0,10 | | - |
| 1-(1,2,3,4,5,6,7,8-octahydro-2,3,8,8,-tetramethyl-2-naphthyl)ethan-1-one | | 0,05 | 0,10 | |
| 3-Methyl-5-phenyl-1-pentanol | 0,05 | | | 0,05 |
| Parfüm | 0,30 | 0,30 | 0,50 | 0,50 |
| Wasser, ad | ad 100,00 | ad 100,00 | ad 100,00 | ad 100,00 |

| **Rezepturbeispiele** | **67** | **68** | **69** | **70** |
|---|---|---|---|---|
| **Chemische/INCI-Bezeichnung** | **Gew.-%** | **Gew.-%** | **Gew.-%** | **Gew.-%** |
| Steareth-100 | 1,00 | 1,00 | 1,00 | 1,00 |
| Polyglyceryl-3 Diisostearat | 1,60 | 1,60 | 1,60 | 1,60 |
| PEG-45/Dodecyl Glykol Copolymer | 0,80 | 0,80 | 0,80 | 0,80 |
| C20-40 Alkyl Stearat | 10,00 | 10,00 | 10,00 | 10,00 |
| Caprylic/Capric Triglycerid | 3,00 | 3,00 | 3,00 | 3,00 |
| Octyldodecanol | 3,00 | 3,00 | 3,00 | 3,00 |
| Dicaprylyl Ether | 4,00 | 4,00 | 4,00 | 4,00 |
| Drometrizol trisiloxan | 2,00 | 1,00 | 2,00 | 1,00 |
| 4-Methylbenzylidencampher | 0,50 | 1,50 | 0,50 | 1,50 |
| Butylenglykol | 4,00 | 4,00 | 4,00 | 4,00 |
| N-(4-(2,4-Dihydroxyphenyl)thiazol-2-yl)-isobutyramid | 0,10 | 0,10 | 0,05 | 0,05 |
| N-(4-(2,4-Dihydroxyphenyl)thiazol-2-yl)-pivalamid | 0,25 | 0,30 | - | 0,05 |
| N-(4-(2,4-Dihydroxyphenyl)thiazol-2-yl)-butyramid | 0,10 | 0,25 | - | 0,10 |
| N-(4-(2,4-Dihydroxyphenyl)thiazol-2-yl)-cyclohexancarboxamid | 0,01 | 0,60 | - | 0,20 |
| Hydroxyisohexyl 3-Cyclohexencarboxaldehyd | 0,05 | 0,05 | 0,05 | 0,05 |
| Parfüm | 0,35 | 0,30 | 0,25 | 0,15 |
| Wasser, ad | ad 100,00 | ad 100,00 | ad 100,00 | ad 100,00 |

### Beispielrezepturen

| **Rezepturbeispiele** | **71** | **72** | **73** | **74** |
|---|---|---|---|---|
| **Chemische/INCI-Bezeichnung** | **Gew.-%** | **Gew.-%** | **Gew.-%** | **Gew.-%** |
| Alkohol denat. | 20,0 | 20,0 | 30,0 | 30,0 |
| Hydroxyethylcellulose | 0,40 | 0,40 | 0,30 | 0,30 |
| Polyethylenglykol 400 | 3,00 | 3,00 | 2,00 | 2,00 |
| Polyethylenglykol (2000) hydriertes Rizinusöl | 2,00 | 2,00 | 3,00 | 3,00 |
| Persea Gratissima Öl (Avokadoöl) | 0,50 | 0,50 | 0,10 | 0,10 |
| 4-[(Cyclopentylhydroxyphenylacetyl)oxy]-1,1-dimethyl-piperidiniumbromid | 0,10 | 0,30 | - | - |
| Homosalate | 1,00 | 1,00 | 2,00 | 1,00 |
| Isoamyl p-Methoxycinnamat | 0,50 | 1,00 | 0,50 | 1,00 |
| Polysilicone-15 | 0,20 | 0,50 | 1,00 | 0,10 |
| Dinatrium Phenyl Dibenzimidazol Tetrasulfonat | 2,00 | 2,00 | 1,00 | 2,00 |
| N-(4-(2,4-Dihydroxyphenyl)thiazol-2-yl)-isobutyramid | 0,10 | 0,10 | 0,05 | - |
| N-(4-(2,4-Dihydroxyphenyl)thiazol-2-yl)-pivalamid | 0,25 | - | 0,01 | 0,05 |
| N-(4-(2,4-Dihydroxyphenyl)thiazol-2-yl)-butyramid | 0,10 | - | 0,15 | - |
| N-(4-(2,4-Dihydroxyphenyl)thiazol-2-yl)-cyclohexancarboxamid | 0,01 | - | 1,00 | 0,20 |
| Coumarin | - | - | 0,05 | - |
| Benzylsalicylat | - | 0,05 | - | - |
| Butylphenylmethylpropional | 0,05 | - | - | - |
| Parfüm | 0,25 | 0,30 | 0,50 | 0,30 |
| Wasser, ad | ad 100 | ad 100 | ad 100 | ad 100 |

| **Rezepturbeispiele** | **75** | **76** | **77** | **78** |
|---|---|---|---|---|
| **Chemische/INCI-Bezeichnung** | **Gew.%** | **Gew.%** | **Gew.%** | **Gew.%** |
| 2-Octyldodecanol | 0,50 | 0,50 | 0,50 | 0,50 |
| 1,2-Propylenglykol | 1,00 | 1,00 | 1,00 | 1,00 |
| 2-Butyloctansäure | 0,25 | - | 0,25 | - |
| Aluminiumchlorhydrat | 2,00 | 3,00 | - | 3,00 |
| N-(4-(2,4-Dihydroxyphenyl)thiazol-2-yl)-isobutyramid | 0,10 | - | 0,10 | 0,05 |
| N-(4-(2,4-Dihydroxyphenyl)thiazol-2-yl)-pivalamid | 0,25 | 0,30 | 0,01 | - |
| N-(4-(2,4-Dihydroxyphenyl)thiazol-2-yl)-butyramid | 0,10 | 0,25 | 0,15 | - |
| N-(4-(2,4-Dihydroxyphenyl)thiazol-2-yl)-cyclohexancarboxamid | 0,01 | 0,60 | 1,00 | - |
| Dinatriumphenyldibenzimidazoltetrasulfonat | 1,00 | 1,00 | 2,00 | 1,00 |
| (2-{4-[2-(4-Diethylamino-2-hydroxy-benzoyl)-benzoyl]-piperazin-1-carbonyl}-phenyl)-(4-diethylamino-2-hydroxy-phenyl)-methanon | 0,50 | 1,00 | 0,50 | 1,00 |
| Linalool | 0,05 | - | 0,05 | 0,05 |
| Coumarin | - | - | 0,05 | - |
| Benzylsalicylat | 0,05 | 0,05 | - | 0,05 |
| Parfüm | 0,10 | 0,20 | 0,40 | 0,20 |
| Ethanol | ad 100 | ad 100 | ad 100 | ad 100 |

Die durch Zusammenmischung der jeweiligen Bestandteile erhaltene flüssige Phase wird mit einem Propan-Butan-Gemisch (2.7) im Verhältnis 39:61 in Aerosolbehälter abgefüllt.

| **Rezepturbeispiele** | **79** | **80** | **81** |
|---|---|---|---|
| **Chemische Bezeichnung** | Gew.- % | Gew.- % | Gew.- % |
| Alkohol denat. | 20,0 | 30,0 | 20,0 |
| Hydroxyethylcellulose | 0,40 | 0,30 | 0,40 |
| Polyethylenglykol 400 | 3,00 | 2,00 | 3,00 |
| Polyethylenglykol (2000) hydriertes Rizinusöl | 2,00 | 3,00 | 2,00 |
| Persea Gratissima Öl (Avokadoöl) | 0,50 | 0,10 | 0,50 |
| 4-[(Cyclopentylhydroxyphenylacetyl)oxy]-1,1-dimethyl-piperidiniumbromid | 0,05 | - | - |
| N-(4-(2,4-Dihydroxyphenyl)thiazol-2-yl)-isobutyramid | 0,10 | 0,10 | 0,05 |
| N-(4-(2,4-Dihydroxyphenyl)thiazol-2-yl)-pivalamid | - | 0,30 | 0,01 |
| N-(4-(2,4-Dihydroxyphenyl)thiazol-2-yl)-butyramid | - | 0,25 | 0,15 |
| N-(4-(2,4-Dihydroxyphenyl)thiazol-2-yl)-cyclohexancarboxamid | - | 0,60 | 1,00 |
| Methylen-bis-benzotriazolyltetramethylbutylphenol | 1,50 | 2,00 | 0,50 |
| Dinatriumphenyldibenzimidazoltetrasulfonat | 2,00 | 0,50 | 0,50 |
| 2-Butyloctansäure | - | 0,10 | - |
| Geraniol | - | 0,05 | - |
| Citronellol | 0,05 | - | - |
| Ethyllinalool | - | - | 0,05 |
| Parfüm | 0,30 | 0,40 | 0,20 |
| Wasser, ad | ad 100 | ad 100 | ad 100 |

| **Rezepturbeispiele** | **82** | **83** | **84** |
|---|---|---|---|
| **Chemische/INCI-Bezeichnung** | **Gew.%** | **Gew.%** | **Gew.%** |
| Glycerinmonostearat | 5,00 | 5,00 | 5,00 |
| Polyethylenglykol(2000) monostearat | 2,00 | 2,00 | 2,00 |
| Stearylalkohol | 3,00 | 3,00 | 3,00 |
| Cyclometicon | 4,00 | 4,00 | 4,00 |
| Paraffinöl | 6,00 | 6,00 | 6,00 |
| Trinatrium EDTA | 0,20 | 0,20 | 0,20 |
| Aluminiumchlorhydrat | 2,50 | 2,50 | 2,50 |
| N-(4-(2,4-Dihydroxyphenyl)thiazol-2-yl)-isobutyramid | 0,10 | 0,10 | 0,05 |
| N-(4-(2,4-Dihydroxyphenyl)thiazol-2-yl)-pivalamid | 0,25 | - | 0,01 |
| N-(4-(2,4-Dihydroxyphenyl)thiazol-2-yl)-butyramid | 0,10 | - | 0,15 |
| N-(4-(2,4-Dihydroxyphenyl)thiazol-2-yl)-cyclohexancarboxamid | 0,01 | - | 1,00 |
| Benzophenon-4 | 0,50 | 0,50 | 1,00 |
| Bis-Ethylhexyloxyphenolmethoxyphenyltriazin | 1,50 | 2,00 | 2,00 |
| 2-Methylpropandiol | 3,00 | 3,00 | 3,00 |
| 2-Ethylhexylglycerinether | 0,50 | 0,50 | 0,50 |
| Benzylsalicylat | - | - | 0,05 |
| Triethylcitrat | - | 0,05 | - |
| Hexalcinnamal | 0,05 | - | - |
| Parfüm | 0,40 | 0,30 | 0,20 |
| Wasser, ad | 100 | 100 | 100 |

### Haar-Shampoos

| **Beispielrezeptur** | **85** | **86** |
|---|---|---|
| **Chemische Bezeichnung** | **Gew.-%** | **Gew.-%** |
| Cocamidopropyl Betain | 2,50 | 2,50 |
| Natrium Laureth Sulfat | 9,00 | 9,00 |
| PEG-40 hydriertes Rizinusöl | 0,50 | 0,50 |
| Polyquaternium-10 | 0,20 | 0,20 |
| PEG-8 | 0,50 | 0,10 |
| Natriumbenzoat | 0,45 | 0,45 |
| Laureth-9 | 2,20 | 2,20 |
| Natrium Salicylat | 0,20 | 0,20 |
| Epsilon-Poly-L-Lysine | - | 0,25 |
| Climbazol | 0,45 | 0,45 |
| Perlglanz | 1,50 | 1,50 |
| Butyl Acrylat/Ethyltrimonium Chlorid Methacrylat/Styrol Copolymer | 2,50 | 1,00 |
| N-(4-(2,4-Dihydroxyphenyl)thiazol-2-yl)-isobutyramid | 0,10 | 0,10 |
| N-(4-(2,4-Dihydroxyphenyl)thiazol-2-yl)-pivalamid | 0,25 | - |
| N-(4-(2,4-Dihydroxyphenyl)thiazol-2-yl)-butyramid | 0,10 | - |
| N-(4-(2,4-Dihydroxyphenyl)thiazol-2-yl)-cyclohexancarboxamid | 0,01 | - |
| Benzophenon-4 | 2,00 | 1,50 |
| Parfum | 0,30 | 0,30 |
| Zitronensäure | q.s. | q.s. |
| Natriumchlorid | q.s. | q.s. |
| Wasser | Ad. 100 | Ad. 100 |

### Antischuppen Shampoos

| **Beispielrezeptur** | **87** | **88** |
|---|---|---|
| **Chemische Bezeichnung** | **Gew.-%** | **Gew.-%** |
| Natrium Laurylethersulfat | 9 | 10 |
| Cocamidopropyl Betain | 4 | 3 |
| Dinatrium PEG-5 Laurylcitrat Sulfosuccinat | - | 1 |
| Verdicker | 0.2 | 0,4 |
| Polyquaternium-10 | 0,3 | 0,1 |
| Guar Hydroxypropyl-Trimonium Chlorid | 0,2 | - |
| Benzophenon-4 | 2,00 | 1,50 |
| Climbazol | - | 0,5 |
| Epsilon-Poly-L-Lysine | 1 | 0,2 |
| Laureth-9 | - | 2 |
| Piroctone Olamin | 1,0 | 0,5 |
| Selensulfid | 0,2 | - |
| Zinkpyrithion | 1,0 | 1,0 |
| Perlglanz | - | 2,5 |
| Trübungsmittel | - | 0,5 |
| PEG-40 hydriertes Rizinusöl | 0,5 | 0,2 |
| N-(4-(2,4-Dihydroxyphenyl)thiazol-2-yl)-isobutyramid | 0,10 | 0,10 |
| N-(4-(2,4-Dihydroxyphenyl)thiazol-2-yl)-pivalamid | 0,25 | - |
| N-(4-(2,4-Dihydroxyphenyl)thiazol-2-yl)-butyramid | 0,10 | - |
| N-(4-(2,4-Dihydroxyphenyl)thiazol-2-yl)-cyclohexancarboxamid | 0,01 | - |
| Natriumsalicylat | 0,3 | 0,2 |
| Natriumbenzoat | 0,25 | 0,3 |
| Natriumchlorid | q.s. | q.s. |
| Zitronensäure | q.s. | q.s. |
| Parfüm | q.s. | q.s. |
| Wasser | ad 100 | ad 100 |

### Haartonik

| **Beispielrezeptur** | **89** | **90** |
|---|---|---|
| **Chemische Bezeichnung** | **Gew.-%** | **Gew.-%** |
| Ethanol | 30,0 | 40,0 |
| Panthenol | 0,2 | 0,1 |
| Tocopheryl Acetate | 0,2 | - |
| Benzophenon-4 | 2,5 | 1,0 |
| C12-13 Alkyl Lactate | 0,2 | 0,1 |
| N-(4-(2,4-Dihydroxyphenyl)thiazol-2-yl)-isobutyramid | 0,10 | 0,10 |
| N-(4-(2,4-Dihydroxyphenyl)thiazol-2-yl)-pivalamid | 0,25 | - |
| N-(4-(2,4-Dihydroxyphenyl)thiazol-2-yl)-butyramid | 0,10 | - |
| N-(4-(2,4-Dihydroxyphenyl)thiazol-2-yl)-cyclohexancarboxamid | 0,01 | - |
| Climbazol | 0,1 | 0,1 |
| PEG-40 hydriertes Rizinusöl | - | 0,3 |
| Parfüm, Konservierungsmittel | q.s. | q.s. |
| Wasser | ad 100 | ad 100 |

## Patentansprüche

1. Wirkstoffkombinationen aus einem oder mehreren Alkylamidothiazolen und einer oder mehreren UV-Filtersubstanzen wobei die UV-Filtersubstanz oder die UV-Filtersubstanzen gewählt wird oder werden aus der Gruppe Butylmethoxydibenzoylmethan, Ethylhexylmethoxycinnamat, Octocrylen, Ethylhexylsalicylat, Phenylbenzimidazolsulfonsäure, Dinatriumphenyldibenzimidazoltetrasulfonat, Benzophenon-3, Drometrizol trisiloxan, Benzophenon-4, Homosalate, Benzol-1,4-di(2-oxo-3-bornylidenmethyl-10-sulfonsäure, Polysilicone-15, Ethylhexyl Triazone, Diethylhexyl-Butamidotriazone, Isoamyl p-Methoxycinnamate, Diethylamino Hydroxybenzoyl Hexyl Benzoate, Methylen-bis-benzotriazolyltetramethylbutylphenol, Bis-Ethylhexyloxyphenolmethoxyphenyltriazin, 4-Methylbenzylidencampher, 2,4-Bis-[5-](dimethylpropyl)benzoxazol-2-yl-(4-phenyl)-imino]-6-(2-ethylhexyl)-imino-1,3,5-triazin und/oder (2-{4-[2-(4-Diethylamino-2-hydroxy-benzoyl)-benzoyl]-piperazin-1-carbonyl}-phenyl)-(4-diethylamino-2-hydroxy-phenyl)-methanon, **dadurch gekennzeichnet, daß** das oder die Alkylamidothiazole folgende Struktur aufweisen: 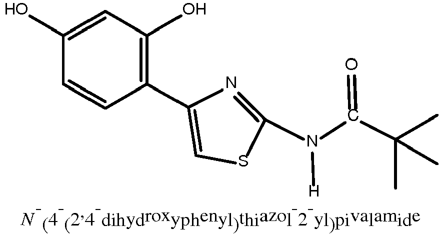 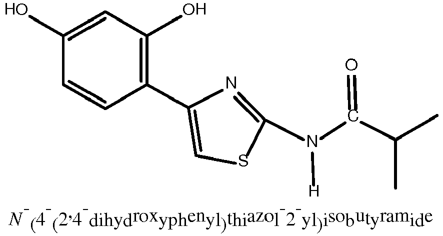 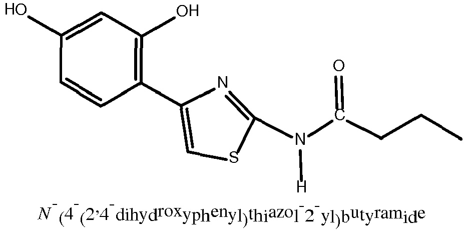 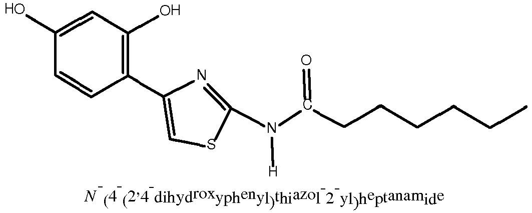 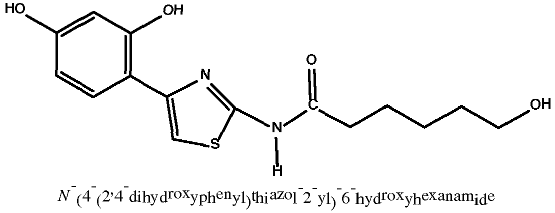 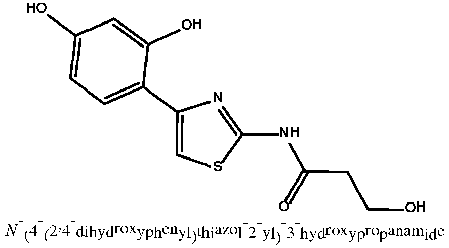 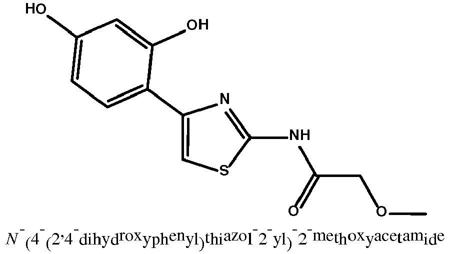 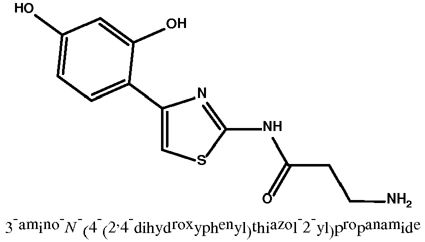 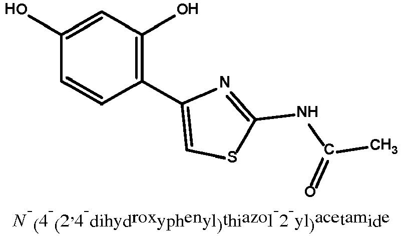 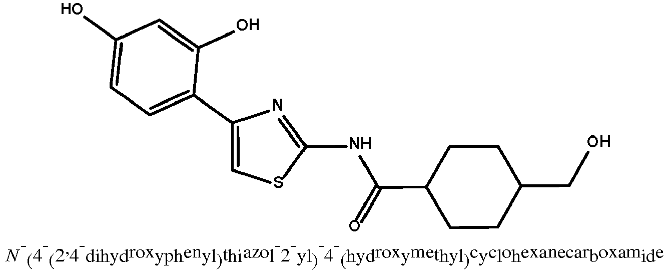 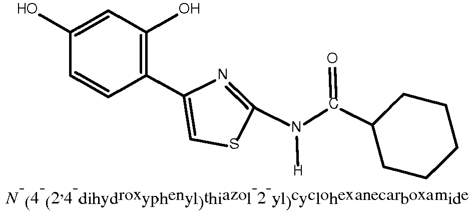 und

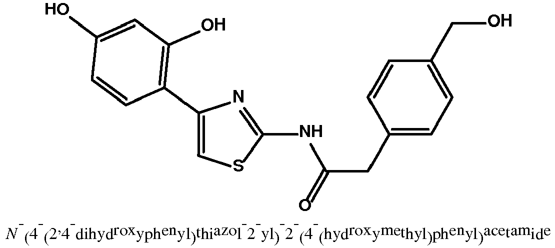

2. Wirkstoffkombinationen nach einem der vorstehenden Ansprüche, **dadurch gekennzeichnet, daß** das oder die Alkylamidothiazole als Halogenid, Carbonat, Ascorbat, , Sulfat, Acetat und/oder Phosphat vorliegen können.

3. Kosmetische oder dermatologische Zubereitungen mit einem Gehalt an Wirkstoffkombinationen nach einem der vorstehenden Ansprüche.

4. Zubereitungen nach Anspruch 3, **dadurch gekennzeichnet, daß** die sie 0,000001 bis 10 Gew.-%, insbesondere 0,0001 bis 3 Gew.-%, ganz besonders 0,001 bis 1 Gew.-% an einem oder mehreren Alkylamidothiazolen enthalten, bezogen auf das Gesamtgewicht der Zubereitung.

5. Zubereitungen nach Anspruch 3, **dadurch gekennzeichnet, daß** die Gesamtmenge der UV-Filtersubstanzen 0,00001 Gew.-% bis 10 Gew.-%, vorzugsweise 0,001 Gew.-% - 5 Gew.-%, insbesondere 0,005 Gew.-% - 3 Gew.-% beträgt, bezogen auf das Gesamtgewicht der Zubereitungen.

6. Wirkstoffkombinationen und Zubereitungen nach einem der vorstehenden Ansprüche zur Verwendung zum Aufhellen der menschlichen Haut.

## Claims

1. Active ingredient combinations of one or more alkylamidothiazoles and one or more UV filter substances, wherein the UV filter substance or the UV filter substances is or are selected from the group butylmethoxydibenzoylmethane, ethylhexyl methoxycinnamate, octocrylene, ethylhexyl salicylate, phenylbenzimidazolesulfonic acid, disodium phenyldibenzimidazoletetrasulfonate, benzophenone-3, drometrizole trisiloxane, benzophenone-4, homosalate, benzene-1,4-di(2-oxo-3-bornylidenemethyl-10-sulfonic acid, polysilicone-15, ethylhexyl triazone, diethylhexyl-butamidotriazone, isoamyl p-methoxycinnamate, diethylamino hydroxybenzoyl hexyl benzoate, methylenebisbenzotriazolyltetramethylbutylphenol, bis-ethylhexyloxyphenolmethoxyphenyltriazine, 4-methylbenzylidenecamphor, 2,4-bis[5-](dimethylpropyl)benzoxazol-2-yl(4-phenyl)imino]-6-(2-ethylhexyl)imino-1,3,5-triazine and/or (2-{4-[2-(4-diethylamino-2-hydroxybenzoyl)benzoyl]piperazine-1-carbonyl}phenyl)-(4-diethylamino-2-hydroxyphenyl)methanone, **characterized in that** the alkylamidothiazole or alkylamidothiazoles have the following structure: 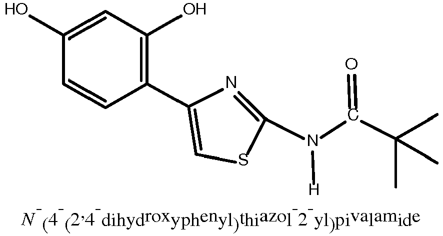 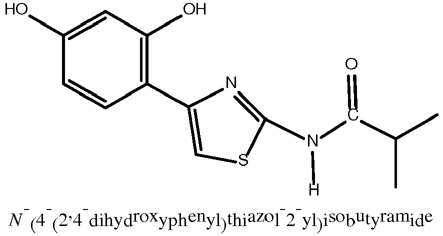 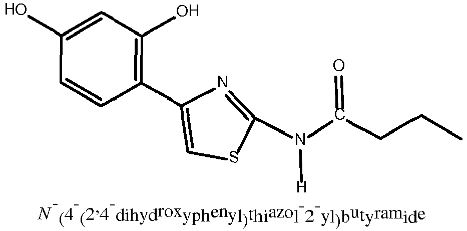 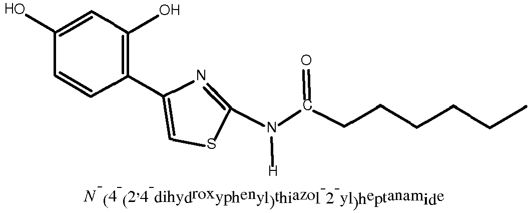 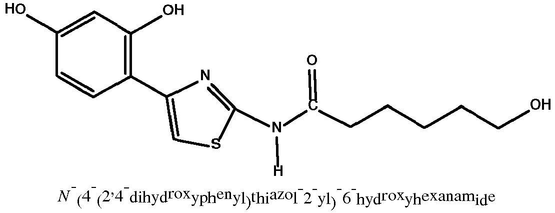 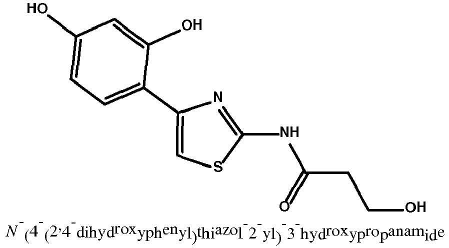 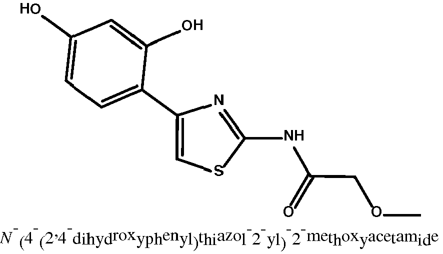 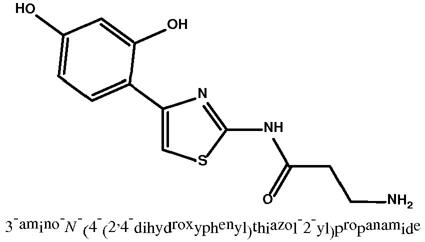 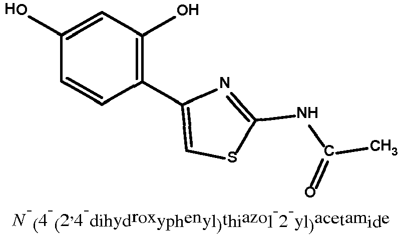 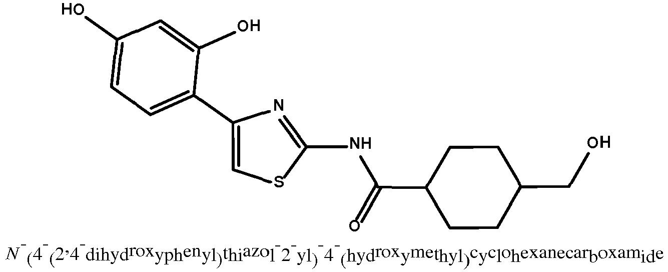 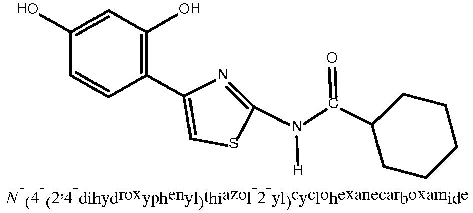 and

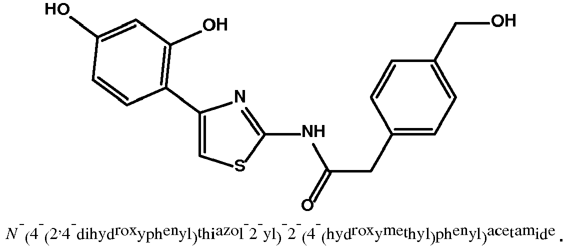

2. Active ingredient combinations according to one of the preceding claims, **characterized in that** the alkylamidothiazole or alkylamidothiazoles can be present as halide, carbonate, ascorbate, sulfate, acetate and/or phosphate.

3. Cosmetic or dermatological preparations with a content of active ingredient combinations according to one of the preceding claims.

4. Preparations according to Claim 3, **characterized in that** the they comprise 0.000001 to 10% by weight, in particular 0.0001 to 3% by weight, very particularly 0.001 to 1% by weight, of one or more alkylamidothiazoles, based on the total weight of the preparation.

5. Preparations according to Claim 3, **characterized in that** the total amount of the UV filter substances is 0.00001 % by weight to 10% by weight, preferably 0.001 % by weight - 5% by weight, in particular 0.005% by weight - 3% by weight, based on the total weight of the preparations.

6. Active ingredient combinations and preparations according to one of the preceding claims for use for the lightening of human skin.

## Revendications

1. Combinaisons d'agents actifs constituées par un ou plusieurs alkylamidothiazoles et une ou plusieurs substances filtrant les UV, la substance filtrant les UV ou les substances filtrant les UV étant choisies dans le groupe constitué par le butylméthoxydibenzoylméthane, le méthoxycinnamate d'éthylhexyle, l'octocrylène, le salicylate d'éthylhexyle, l'acide phénylbenzimidazole-sulfonique, le tétrasulfonate de phényldibenzimidazole disodique, la benzophénone-3, le drométrizol-trisiloxane, la benzophénone-4, l'homosalate, l'acide benzène-1,4-di(2-oxo-3-bornylidène-méthyl-10-sulfonique), la polysilicone-15, l'éthylhexyltriazone, la diéthylhexyl-butamidotriazone, le p-méthoxycinnamate d'isoamyle, le benzoate de diéthylaminohydroxybenzoyl-hexyle, le méthylène-bis-benzotriazolyltétraméthylbutylphénol, la bis-éthylhexyloxyphénolméthoxyphényltriazine, le 4-méthylbenzylidène-camphre, la 2,4-bis-[5-](diméthylpropyl)benzoxazol-2-yl-(4-phényl)-imino]-6-(2-éthylhexyl)-imino-1,3,5-triazine et/ou la (2-{4-[2-(4-diéthylamino-2-hydroxy-benzoyl)-benzoyl]-pipérazine-1-carbonyl}-phényl)-(4-diéthylamino-2-hydroxy-phényl)-méthanone, **caractérisées en ce que** le ou les alkylamidothiazoles présentent la structure suivante : 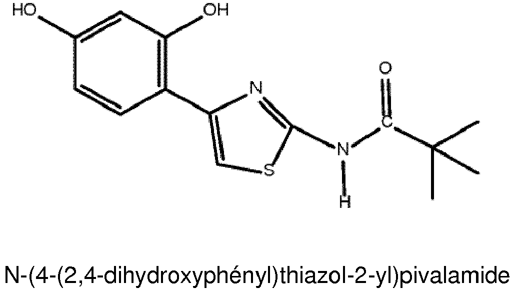 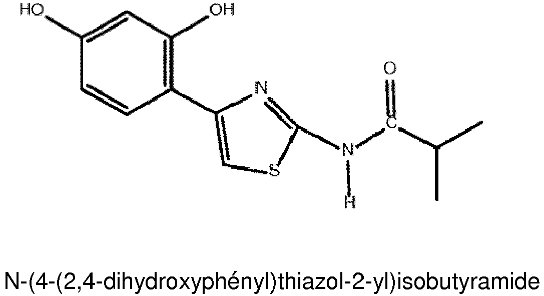 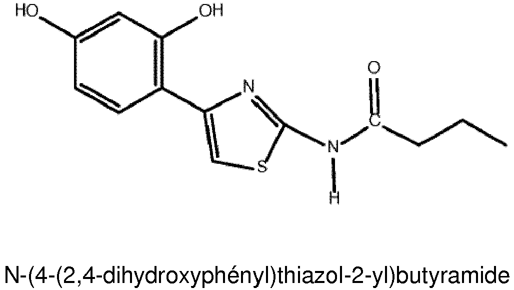 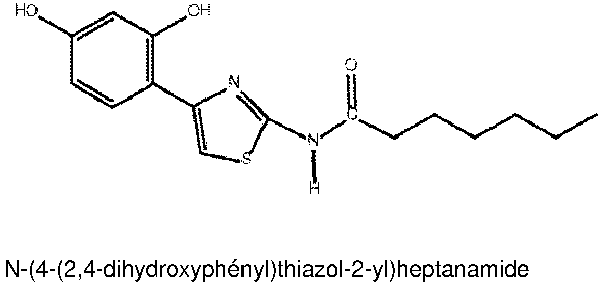 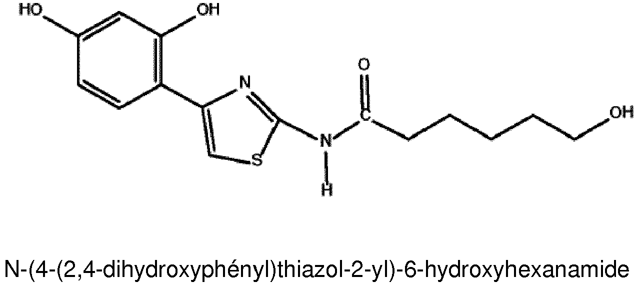 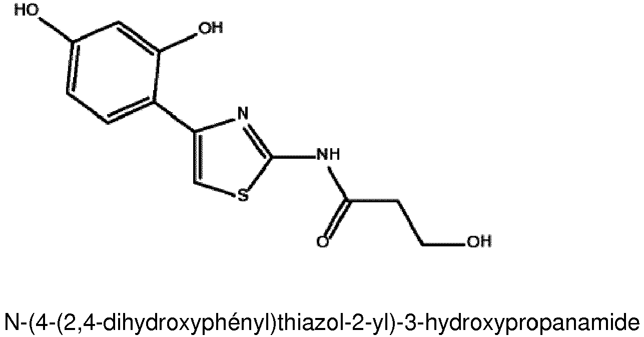 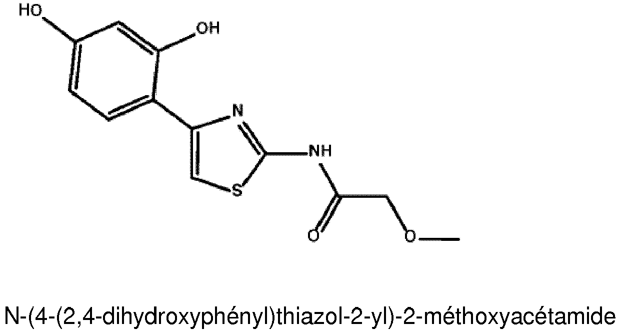 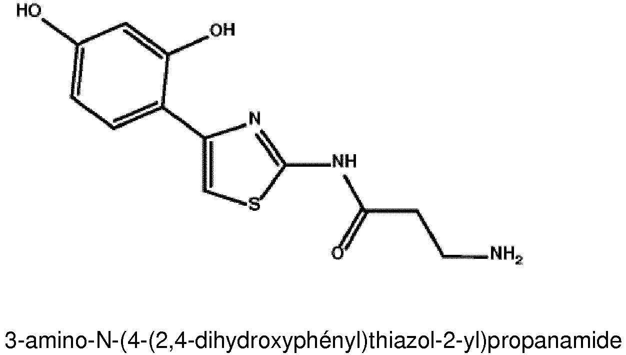 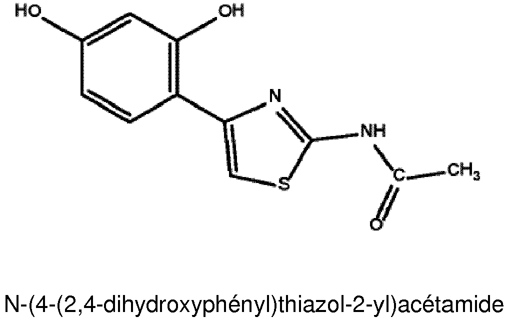 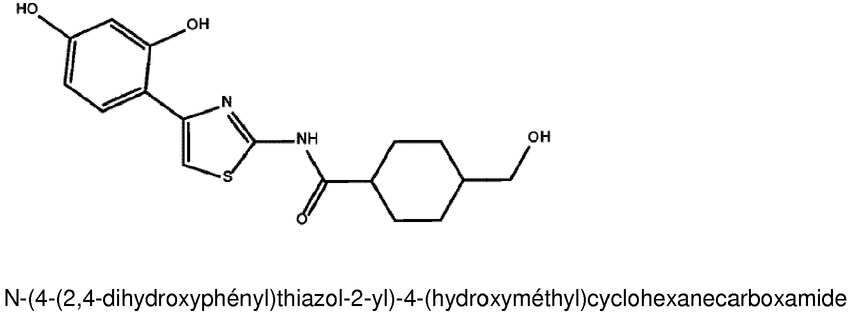 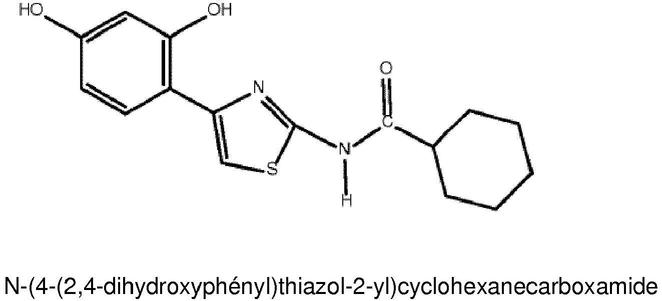 et

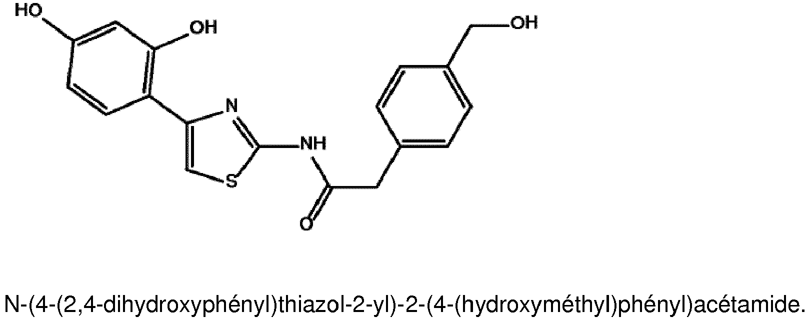

2. Combinaisons d'agents actifs selon l'une quelconque des revendications précédentes, **caractérisées en ce que** le ou les alkylamidothiazoles peuvent se présenter sous la forme d'un halogénure, d'un carbonate, d'un ascorbate, d'un sulfate, d'un acétate et/ou d'un phosphate.

3. Préparations cosmétiques ou dermatologiques ayant une teneur en combinaisons d'agents actifs selon l'une quelconque des revendications précédentes.

4. Préparations selon la revendication 3, **caractérisées en ce que** les elles contiennent 0,000001 à 10 % en poids, notamment 0,0001 à 3 % en poids, tout particulièrement 0,001 à 1 % en poids, d'un ou de plusieurs alkylamidothiazoles, par rapport au poids total de la préparation.

5. Préparations selon la revendication 3, **caractérisées en ce que** la quantité totale de substances filtrant les UV est de 0,00001 % en poids à 10 % en poids, de préférence de 0,001 % en poids à 5 % en poids, notamment de 0,005 % en poids à 3 % en poids, par rapport au poids total des préparations.

6. Combinaisons d'agents actifs et préparations selon l'une quelconque des revendications précédentes, destinées à une utilisation pour l'éclaircissement de la peau humaine.
